Europäisches Patentamt

European Patent Office

Office européen des brevets

(11) Publication number: **0 419 666 A1**

(12) EUROPEAN PATENT APPLICATION
published in accordance with Art.
158(3) EPC

(21) Application number: 90901019.1

(22) Date of filing: 28.12.89

(86) International application number:
PCT/JP89/01330

(87) International publication number:
WO 90/07581 (12.07.90 90/16)

(51) Int. Cl.5: **C12N 15/63**, C12N 15/83,
C12N 7/01

(30) Priority: 29.12.88 JP 335605/88
28.03.89 JP 176025/89

(43) Date of publication of application:
**03.04.91 Bulletin 91/14**

(84) Designated Contracting States:
**DE FR IT**

(71) Applicant: NIPPON ZEON CO., LTD.
6-1, Marunouchi 2-chome
Chiyoda-ku Tokyo 100(JP)

(72) Inventor: OGAWA, Ryohei
2-15-33, Isogo Isogo-ku
Yokohama-shi Kanagawa 235(JP)
Inventor: YANAGIDA, Noboru
480-1, Miyauchi Nakahara-ku
Kawasaki-shi Kanagawa 211(JP)
Inventor: SAEKI, Sakiko
1-16-8, Higashiyaguchi Ota-ku
Tokyo 146(JP)
Inventor: OHKAWA, Setsuko 17-13,
Shinoharanishimachi
Kohoku-ku Yokohama-shi
Kanagawa 222(JP)

(74) Representative: Vossius & Partner
Siebertstrasse 4 P.O. Box 86 07 67
W-8000 München 86(DE)

(54) PROMOTER, PLASMID CONTAINING THE SAME, AND RECOMBINANT AVIPOXVIRUS.

(57) A DNA fragment having a promoter action is isolated from a genomic DNA of a virus related to fowl pox virus (FPV) and its base sequence is determined. An efficient manifestation of an exogenous gene in a host cell becomes possible by introducing a chimera gene formed by joining the DNA having a promoter action with an exogenous gene into a host cell. Furthermore an infective recombinant pox virus which manifests an exogenous gene efficiently by homologous recombination can be obtained by introducing a recombinant plasmid comprising a chimera gene inserted into the DNA region derived from the pox virus into a cell infected with the pox virus. The present invention thus makes it possible to mass-produce vaccine in a stabilized manner.

# PROMOTER, PLASMID CONTAINING THE SAME AND RECOMBINANT AVIPOXVIRUS

## TECHNICAL FIELD

The present invention relates to a DNA sequence capable of efficiently expressing an exogenous gene in poxvirus genome by functionally binding to the exogenous gene, a method for selection of the DNA sequence, a chimera gene comprising the DNA sequence functionally ligated with the exogenous gene, a plasmid bearing the chimera gene and infectious recombinant poxvirus containing such a chimera gene.

## BACKGROUND ART

In recent years, fowlpoxvirus (hereafter abbreviated as FPV) was analyzed by means of molecular biology [Fiona Tomley et al., J. Gen Virol., 69 , 1025-1040 (1988); R. Drillen et al., Virology, 160 , 203-209 (1987); Matthew M. Binns et al., J. Gen. Virol., 69 , 1275-1283 (1988)]. However, only several genes have been identified specifically [David B. Boyl et al., Virology, 156 , 355-365 (1987); David B. Boyl et al., J. Gen. Virol., 67 , 1591-1600 (1988); Matthew M. Binns et al., Nucleic Acid Research, 15 , 6563-6573 (1987)].

Recently, recombinant FPV was reported for the first time [David B. Boyl et al., Virus Research, 10 , 343-356 (1988)], where 7.5K promoter of vaccinia virus and PLII promoter are used as transcription-regulatory sequences for expression of an exogenous gene.

Recombinant FPV is expected to be effective as a polyvalent live vaccine, similarly to recombinant vaccinia virus. In order to use as live vaccine of trivalency or more, however, it is necessary to simultaneously express at least two exogenous genes in FPV genome. For concurrent expression of a plurality of exogenous genes, there are basically three methods. A first method comprises ligating exogenous genes, respectively, under control of a sequence having one promoter activity and inserting them into different recombination sites. In this case, a plurality of recombination sites are required. A second method comprises inserting many exogenous genes into one recombination site. In this method, a sequence having one promoter activity may express a plurality of exogenous genes, depending on the number or length of exogenous gene or way of ligation. However, in view of an expression efficiency, it is desired to use one sequence having a promoter activity per one exogenous gene. A third method which is most preferred comprises ligating a plurality of exogenous genes at a plurality of recombination sites, respectively, under nucleotide sequences having different promoter activities.

Homologous sequence of about 100 bp causes spontaneous recombination of DNA. Therefore, where the inserted sequence on genome is located as closely to a sequence having high homology to that of about 100 bp in the sequence, deletion of the inserted exogenous gene ligated to the sequence tends to occur. That is, in the case of using a plurality of sequences each having one promoter activity, the deletion tends to occur when sequences having the respective promoter activities are located closely to each other. Particularly where a plurality of exogenous genes are inserted into one site together with a sequence having the same promoter activity, its tendency is remarkable; however, even where the exogenous genes are inserted into many recombination sites, the tendency is noted. For this reason, it was difficult to insert many exogenous genes.

Tomley et al. recently determined the sequence of Bam HI fragment of FPV having about 11.2 kbp and identified about 20 open reading frames. In some of the upstream sequences, a consensus sequence found in promoters of vaccinia virus or a sequence similar thereto is recognized, suggesting that the sequence would be a promoter [Fiona Tomely et al., J. Gen. Viol., 69 , 1025-1040 (1988)].

However, it is unclear that these sequences have a promoter activity; even conceded that the sequences would have a promoter activity, it is unclear as to if these sequences could sufficiently express exogenous genes in recombinant virus.

## DISCLOSURE OF THE INVENTION

Under the state of art, the present inventors have made extensive studies with an attempt to develop a promoter capable of efficiently express the incorporated exogenous genes in FPV genome. As a result, it has been found that by selecting a sequence having a promoter activity from sequences derived from virus akin to FPV and functionally ligating the sequence with exogenous genes, the sequence could be stably expressed in FPV genome in which a plurality of exogenous genes have been inserted, independently or in

EP 0 419 666 A1

combination. The present invention has thus been attained.


BRIEF DESCRIPTION OF THE DRAWINGS

Figs. 1, 2, 3 and 4 show nucleotide sequences of fragments 14-6, 49-22, 41-3 and 37-1 having the promoter activity according to the present invention.

Fig. 5 shows a nucleotide sequence of about 100 bp from Bam HI cleavage site of pNZ131-derived fragment of about 3.1 kbp.

Fig. 6 shows a nucleotide sequence of hemagglutinin neuraminidase gene of Newcastle disease virus.

Fig. 7 and Fig. 8 show restriction enzyme maps of pNZ131 and pNZ1127R, respectively.

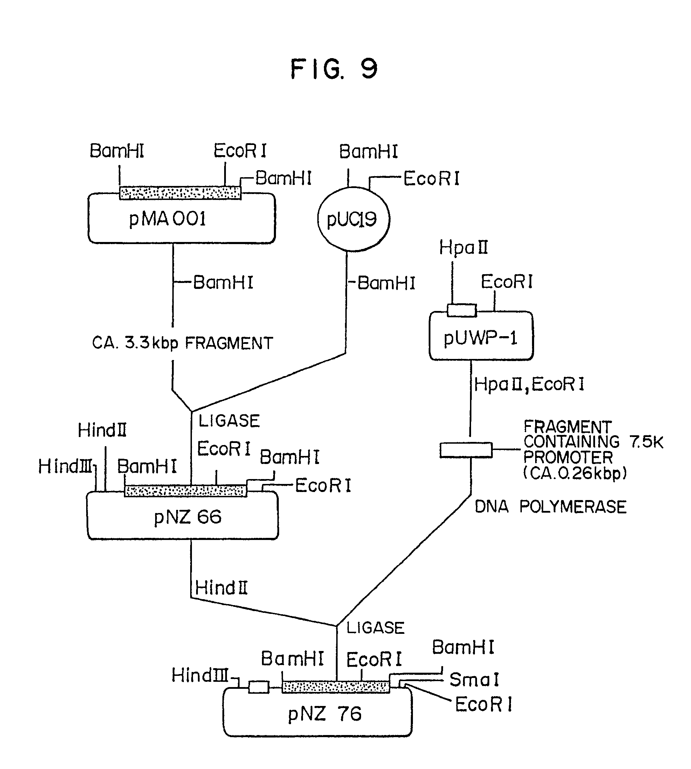

Figs. 9, 10, 11, 12, 13, 14 and 15 indicate procedures for construction of pNZ76, pNZ1127R, mp10-HN180, pNZ136S, pNZ136SL, pNZ2437R and pNZ2127RL, respectively.

Fig. 16 shows the synthetic DNA sequence.

Figs. 17, 18 and 19 indicate procedures for construction of pNZ2537L, pNZ253717L15 and pNZ2637, respectively.


BEST MODE FOR PRACTICING THE INVENTION

According to the present invention, there is provided a DNA sequence capable of expressing exogenous genes in Avipoxvirus genome under its control.

In order to obtain a nucleotide sequence having the promoter activity of the present invention, a DNA fragment is inserted into a plasmid carrying a marker gene that is not under control of the promoter, at the upstream of the marker gene, by shotgun cloning. Among cells infectd with Avipoxvirus (hereafter abbreviated as APV), a cell which has come to express the marker gene is selected and a sequence of the inserted fragment may be provided for analysis.

A gene which can be used as the marker gene in the present invention is not particularly limited, so far as its expression can be confirmed, but a gene, which expression is readily confirmed, is preferably used. A specific example of such a gene is lacZ gene. When lacZ gene is expressed, chlorophenol red-$\beta$-D-galactopyranoside (hereafter abbreviated as CPRG) is decomposed by $\beta$-galactosidase to form a red color so that detection can be readily made. When a plasmid in which lacZ gene has been inserted at the downstream site thereof where no promoter is present is transfected to APV-infected cells which do not substantially show $\beta$-galactosidase activity, no color formation is noted. When a DNA fragment is inserted into the plasmid at the upstream of lacZ gene and transfected to the same cells to form a red color, the inserted DNA fragment obviously has promoter activity. By analysis of the nucleotide sequence, the nucleotide sequence having the promoter activity of the present invention can be obtained.

The nucleotide sequence having the promoter activity in the present invention may be of any origin but poxvirus is preferable. Among them, Avipoxvirus (hereafter abbreviated as APV), especially FPV or a virus used as avian pox live vaccine akin to FPV is preferred. Examples of virus used as avian pox live vaccine akin to FPV are chick embryo habituated pigeonpoxvirus Nakano strain (manufactured by Nippon Pharmacy Co., Ltd.; hereafter abbreviated as NP strain) and the like.

Where recombinant APV incorporated with a nucleotide sequence eventually having the promoter activity is prepared, it is recommended to incorporate the marker gene into the non-essential APV DNA site of a plasmid incorporated with a genome region non-essential to proliferation of APV and insert a candidate of the nucleotide sequence having the promoter activity at the upstream thereof.

The chimera gene of the present invention ligated with an exogenous gene at the downstream of the nucleotide sequence having the promoter activity is incorporated into genome or transfected to APV-infected cells as a plasmid, whereby the exogenous gene can be expressed. The exogenous gene is not particularly limited; as such a gene that makes use of the characteristic feature of the present invention, there may be exemplified a gene encoding antigen protein of pathogenic virus, etc., e.g., antigen protein of Newcastle disease virus, especially represented by hemagglutinin neuraminidase.

cDNA encoding hemagglutinin neuraminidase (hereafter abbreviated as HN) derived from Newcastle disease virus (hereafter abbreviated as NDV) can be prepared using, for example, D-26 strain of NDV or Beaudette C strain.

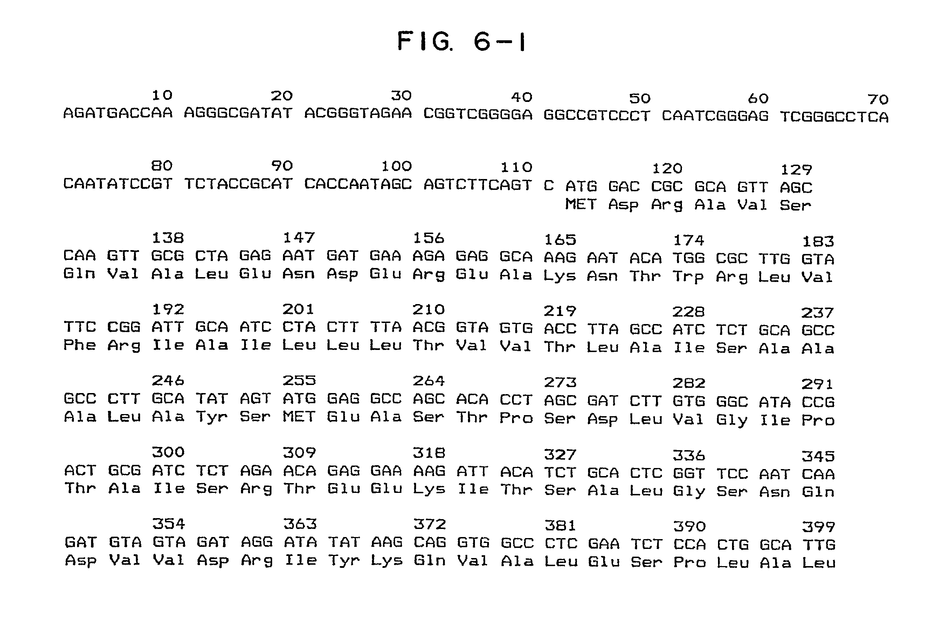

cDNA prepared from, e.g., D-26 strain includes two types. One is composed of 1746 base pairs from 12 to 1857 and another is composed of 1848 base pairs from 112 to 1959, in the nucleotide sequence shown in Fig. 5. A difference between these cDNAs lies in the 1747th nucleotide in Fig. 5 (nucleotide marked with *

3

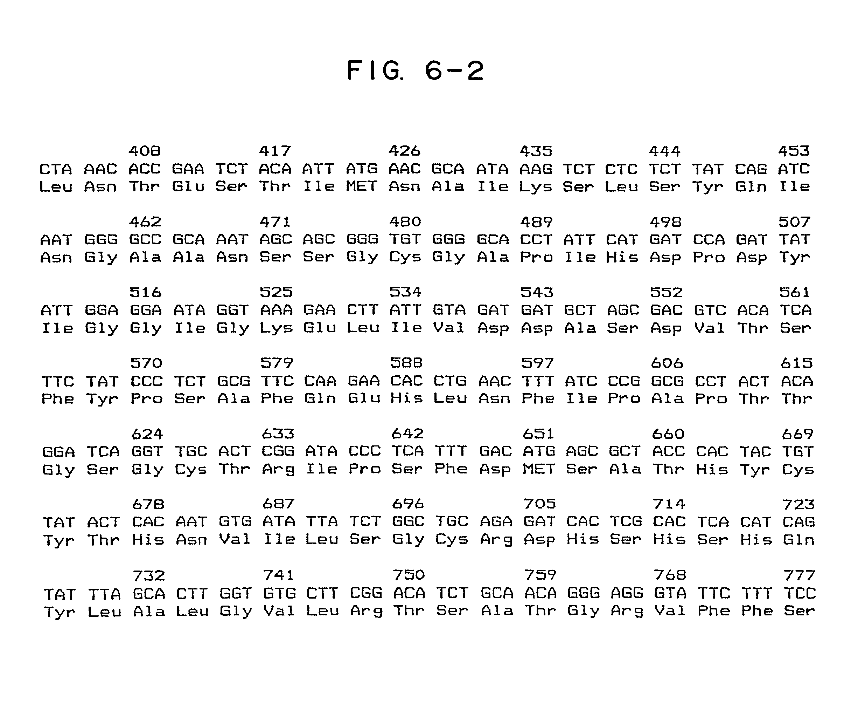

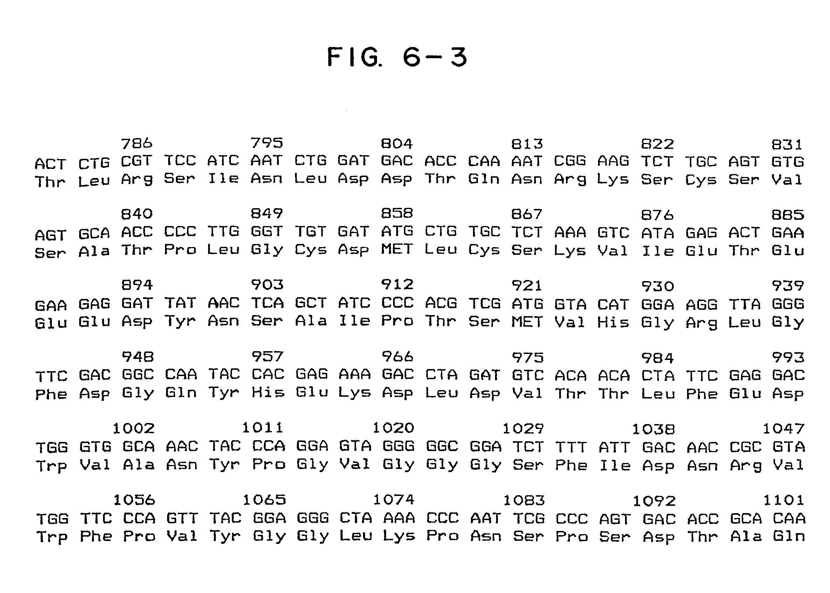

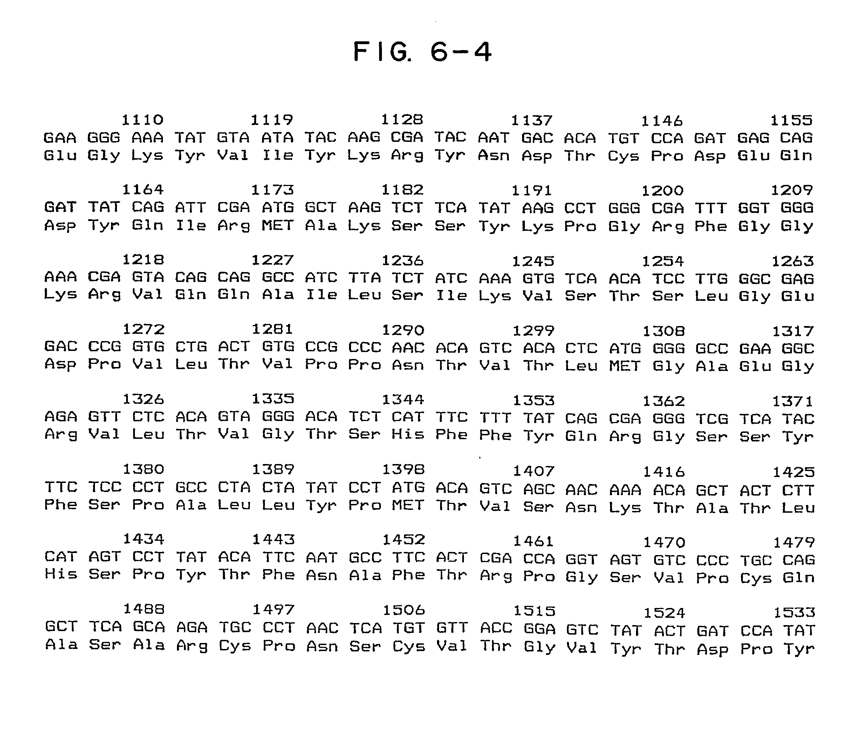

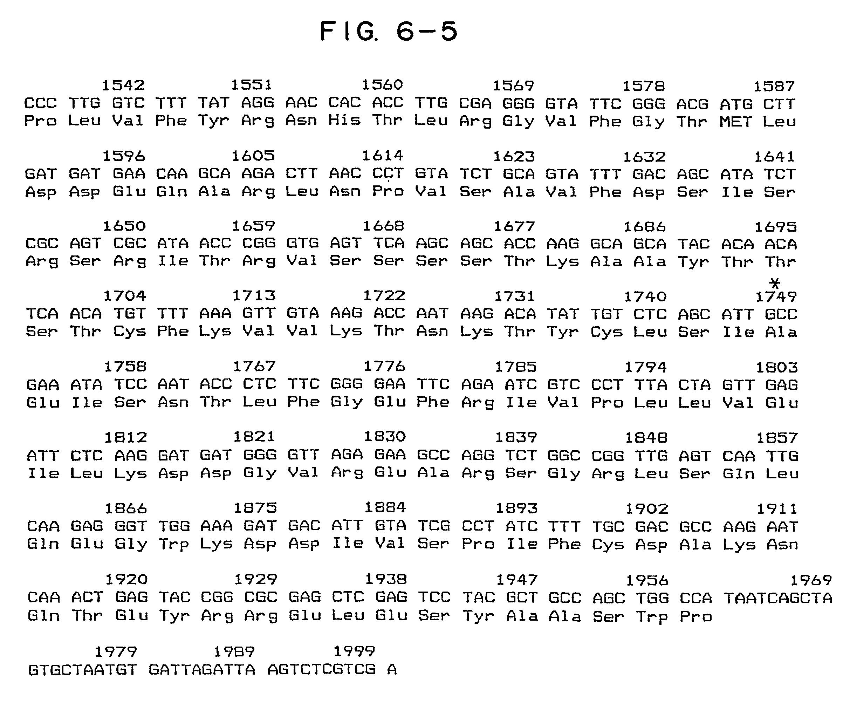

in Fig. 6). The latter cDNA is C as illustrated in the figure but in the former, C is replaced by T and as the result, translation termination codon (TAA) is formed. cDNA used in the examples later described is the former one which has a size shorter than that of the latter.

Furthermore, cDNA prepared from Beaudette C strain is composed of 1731 base pairs [N. Miller, J. Gen. Virol., 67 , 1917-1927 (1986)] and has a structure in which even 170 nucleotides are substituted, when the structure is compared to that of cDNA prepared from D-26 strain. As stated above, sequences of cDNA encoding NDV-derived HN gene are not always the same; however, as long as cDNA has a nucleotide sequence which is substituted, inserted or deleted within such a range as retaining substantially the same function as that of the above three cDNA, such cDNA may be used without causing any inconvenience. Of course, its amino acid sequence may also be modified to a different degree, so long as cDNA has substantially the same function.

The plasmid of the present invention which carries a nucleotide sequence having the promoter activity and chimera gene ligated to an exogenous gene under its control can express the exogenous gene, when the plasmid is transfected to APV-infected cells.

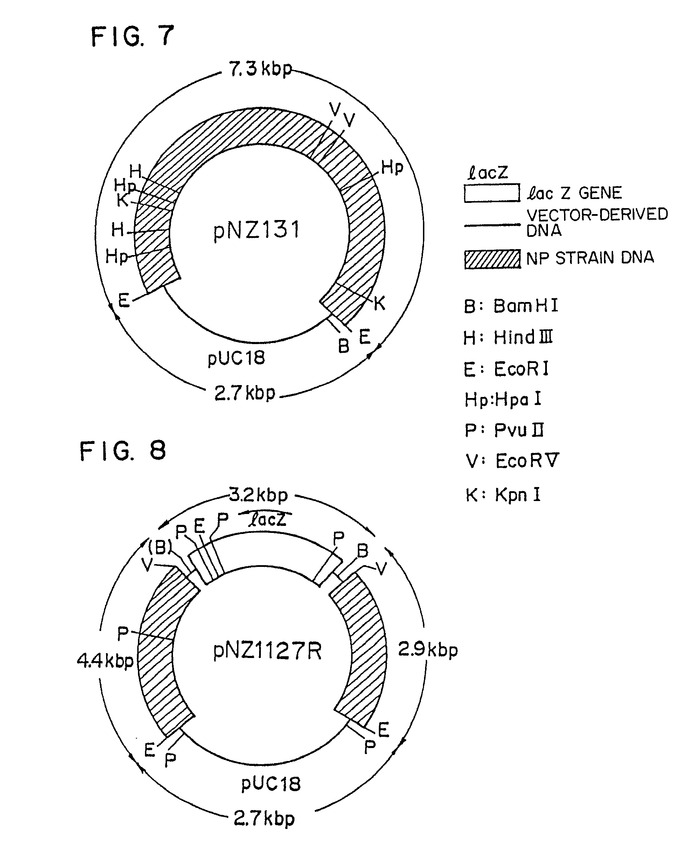

Further where the chimera gene in this plasmid is inserted into the nucleotide sequence derived from genome region non-essential to proliferation of APV, transfection of this plasmid to cultured animal cells infected with APV causes homologous recombination between virus genome DNA and APV-derived DNA in the plasmid to construct recombinant APV. A specific example of the genome region non-essential to proliferation includes a region which causes homologous recombination with Eco RI fragment (about 7.3 kbp) of NP strain DNA showing a restriction enzyme map as given in Fig. 7; etc. The animal culture cells used in this step may be any cells which can be infected with APV to proliferate. Specific examples of the animal culture cells are chicken-derived culture cells such as chick embryo fibroblast, etc. and chick chorio-allantoic membrane, etc.

Construction of the recombinant APV may be carried out in a conventional manner. A recombinant vector is transfected by the calcium phosphate coprecipitation method or by the electroporation method, to host cells infected with Avipoxvirus according to, for example, D.M. Glover, DNA Cloning, volume II, A Practical Approach, pp. 191-211, IRL Press, Oxford, Washington. The thus obtained virus mass containing recombinant virus is infected to host cells cultured on Eagle's MEM. Plaques grown on the medium may be made recombinant virus candidate strains. For selection of a virus having inserted therein the DNA fragment encoding HN of NDV from these candidate strains, the candidate strains may be selected and purified by a hybridization method using the DNA as a probe and confirmed by immunoassay using an anti-NDV antibody.

In order to enhance a dose of the exogenous gene expressed, a transcription termination factor may be introduced into the exogenous gene at its downstream. The transcription termination factor introduced and a method for the introduction are not limited, so long as they are factor and method usable for host.

Where the same virus as that used for selection of a nucleotide sequence having the promoter activity or a virus akin thereto is used for the recombination, a site having high homology to a nucleotide sequence having the promoter activity is present adjacent the recombination site and becomes a cause for deletion so that stable recombinant APV cannot be prepared in some occasion. For this reason, in case that an expression dose of the incorporated exogenous gene decreases and comes into problem, the problem may be solved by utilization of other recombination site, utilization of a nucleotide sequence having other promoter activity or by incorporation of the exogenous gene to be expressed into a direction opposite the site having high homology to the nucleotide sequence having the promoter activity which is located adjacent the recombination site; etc. In a certain case, the nucleotide sequence may be controlled to have such a size that causes no homologous recombination is caused, by shortening the nucleotide sequence in such a range as having the promoter activity of required potency, such as removal of a portion, which less affects the promoter activity, from the nucleotide sequence having the promoter activity; etc.

As the portion removed for shortening which less affects the promoter activity, there may be exemplified an open reading frame under control of the promoter activity of its sequence; etc. According to the current technique for reading genetic code, however, it is often difficult to determine which portion would be necessary for the promoter activity. It is thus eventually necessary to shorten the nucleotide sequence by removing nucleotides one by one, while confirming if the nucleotide has a promoter activity of required potency. The promoter activity of required potency as used herein refers to a promoter activity sufficient to provide an expression dose required for cells in which the exogenous gene is to be expressed.

In addition to the portion which is necessary at least for exhibiting the promoter activity as described above, the nucleotide sequence having the promoter activity may sometimes contain a portion which enhances the promoter activity when it is removed, or a portion causing no reduction in the promoter activity even though it is removed. While monitoring deletion caused by homologous recombination or an expression dose of the exogenous gene, etc., these additional portions are removed or, if necessary or desired, added to obtain the nucleotide sequence having the most preferred promoter activity, depending on purpose.

Therefore, according to the present invention, there are provided a plurality of novel Avipoxvirus promoters capable of expressing polypeptide in the recombinant virus, which can lead to construction of infectious recombinant poxvirus as polyvalent vaccine having at least trivalency which can stably express large varieties of exogenous genes.

[Examples]

Hereafter the present invention is described in more detail, by referring to the examples below.

Example 1

Identification of DNA fragment having promoter activity from NP strain

(1) Preparation of DNA from NP strain genome:

NP strain of about $3.0 \times 10^7$ plaque forming unit (PFU) was infected to chick embryo fibloblasts cultured in a 150 $cm^2$ culture flask. After culturing at 37°C in 5% $CO_2$ incubator for 72 hours, the procedure of freezing and thawing was repeated 3 times to prepare a stock solution. The stock solution was centrifuged at 3000 r.p.m. for 10 minutes to remove cell debris. The supernatant was further centrifuged at 25000 rpm for 60 minutes and the precipitated virus particles were recovered. The virus particles were suspended in 800 $\mu l$ of buffer (50 mM Tris-HCl, 1 mM $MgCl_2$, pH 7.5) containing 15 $\mu g$ of DNase (manufactured by Takara Shuzo Co., Ltd.) followed by incubation at 37°C for an hour. Then 100 $\mu l$ of 250 mM EDTA solution was added and the mixture was allowed to stand at room temperature for 30 minutes. Thereafter, 100 $\mu l$ of 10% SDS solution and 50 $\mu l$ of 0.1% proteinase K (manufactured by Boehringer Mannheim Co., Ltd.) were added to the mixture followed by reacting at 50°C for an hour. Subsequently, TE (1 mM EDTA, 10 mM Tris-HCl aqueous solution, pH 8.0) was added to an equal volume mixture of phenol and chloroform until the aqueous phase was separated from the organic solvent phase and, 1 ml of the organic solvent phase was added to the reaction mixture described above to gently suspend the reaction mixture in the organic solvent phase. After allowing to stand for 10 minutes, the suspension was centrifuged at 3500 rpm for 10 minutes to separate into two phases. The aqueous phase which formed the upper layer was recovered and the protein was removed. Further 2 ml of ethanol was added to precipitate virus DNA. The precipitated DNA was washed with 70% ethanol and dried to give about 500 ng of NP strain-derived DNA.

(2) Construction of plasmid pNZ76 (cf. Fig. 8):

About 0.26 kbp of Sal I-Rsa I fragment containing promoter of DNA encoding 7.5 K dalton peptide of vaccinia virus WR strain (hereafter abbreviated as 7.5 K promoter) was incorporated into pUC9 at the Sal I-Sma I site. The resulting plasmid was named pUWP-I.

After digesting 10 $\mu g$ of pMA001 [Shirakawa et al., Gene, 28, 127 (1984)] with Bam HI, $\beta$-galactosidase gene (about 3.3 kbp) was recovered. On the other hand, after digesting 0.3 $\mu g$ of pUC19 with Bam HI, extraction was performed with phenol-chloroform (1 : 1) and recovery was made through ethanol precipitation. By ligation with the $\beta$-galactosidase gene prepared, hybrid plasmid pNZ66 was constructed.

On the other hand, 40 $\mu g$ of pUWP-I was digested with Hpa II and Eco RI and, a fragment of about 0.26 kbp containing 7.5 K promoter was separated by 1.5% low melting point agarose gel electrophoresis (70 volts, 6 hours) and was made the blunt end by DNA polymerase. After 0.3 $\mu g$ of pNZ66 was digested with Hinc II, extraction was performed with phenol-chloroform (1 : 1). By ligation with the 7.5 K promoter gene of about 0.26 kbp described above, a hybrid plasmid was obtained and it was named pNZ76.

pNZ76 is a plasmid containing the DNA fragment ligated with 7.5 K promoter of vaccinia virus and lacZ gene.

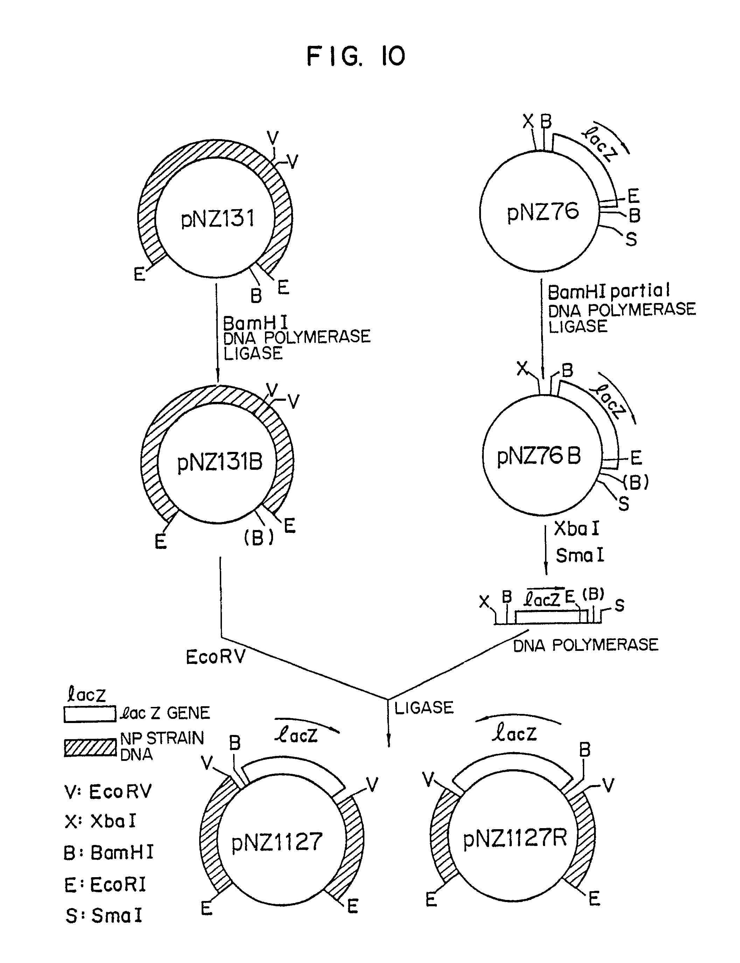

(3) Construction of promoter probe vector pNZ1127R (cf. Fig. 10)

NP strain-derived DNA (1 $\mu$g) prepared in (1) was digested with Eco RI. The digestion product was mixed with 500 ng of pUC18 (manufactured by Pharmacia Inc.) digested with Eco RI and was ligated using T4 DNA ligase to give many clones.

Among them, a clone which was ligated with about 7.3 kbp NP strain DNA fragment shown in Fig. 7 was named pNZ131.

After digesting 1 $\mu$g of pNZ131 with Bam HI, was made the blunt end by Klenow fragment and again ligated using T4 DNA ligase to change a sequence of the Bam HI recognition site, thus incapable of being recognized with Bam HI. The clone was named pNZ131B.

Further pNZ76 was partially cleaved with Bam HI and rendered the blunt end followed by ligation again. A sequence was changed in one Bam HI cleavage site positioned at the downstream of lacZ gene of the two cleavage sites so that the plasmid was not recognized with Bam HI. The plasmid was doubly cleaved with Eco RI and Bam HI and selected by applying to 0.8% agarose gel electrophoresis, which was named pNZ76B. After digesting pNZ76B with Xba I and Sma I, about 3.2 kbp DNA fragment containing lacZ gene was extracted and changed to the blunt end by Klenow fragment.

pNZ131B digested with Eco RV was mixed with about 3.2 kbp fragment described above. The mixture was ligated using T4 DNA ligase to construct plasmids pNZ1127 and pNZ1127R (Fig. 8).

(4) Construction of recombinant virus (transfection)

NP strain of about 1.5 x $10^7$ p.f.u. was infected at 0.05 m.o.i. to chick embryo fibloblasts (hereafter abbreviated as CEF) cultured in an incubator at 37°C in 5% $CO_2$ overnight in a 75 cm² culture flask. Two hours after, 15 ml of CEF growth medium (Eagle's MEM containing 5% fetal calf serum, 0.03% L-glutamine and 10% Tryptose phosphate broth) was added thereto followed by incubation for further 2 hours. Then the medium was discarded and the cells were washed twice with 10 ml of Dulbecco-PBS (-) (manufactured by Nissui Pharmaceutical Co., Ltd.) incubated at 37°C and, 400 $\mu$l of 0.1% Trypsin (manufactured by Difco Co.) was added to recover the cells.

After the cells were suspended in 2 ml of Dulbecco-PBS (-), the suspension was centrifuged at 1500 rpm for 5 minutes and Dulbecco-PBS (-) was then removed. Further after suspending in 20 ml of Saline G (0.8% NaCl, 0.04% KCl, 0.0395% $Na_2HPO_4.12H_2O$, 0.02% $KH_2PO_4$, 0.01% $MgCl_2.6H_2O$, 0.01% $CaCl_2$, 0.1% glucose, pH 7.1), the suspension was centrifuged at 1500 rpm for 5 minutes to remove Saline G. Then, the precipitated cells were suspended in 700 $\mu$l of Saline G and the suspension was transferred to a cuvette for electroporation.

A solution containing 10 $\mu$g of pNZ1127 was fractionated. After ethanol precipitation and washing, it was dried and the plasmid DNA was then dissolved in 100 $\mu$l of Saline G.

After 100 $\mu$l of 100 $\mu$g/ml pNZ1127 solution in Saline G was added to the NP strain-infected cell suspension charged in the cuvette for electroporation, a pulse of 3 kv/cm was applied with Gene Pulser (Biorad Co.). Thereafter, the cell suspension was allowed to stand at room temperature for 10 minutes and transferred to a 25 cm² culture flask charged with 5 ml of CEF growth medium. After culturing at 37°C for 72 hours in a 5% $CO_2$ incubator, the procedure of freezing and thawing was repeated 3 times. The product was made a recombinant virus stock. With respect to pNZ1127R, the same procedures were performed to give a recombinant virus stock.

(5) Plaque assay

After 1.0 x $10^7$ of CEF cultured in 10 cm Petri dish overnight was washed twice with Eagle's MEM, 500 $\mu$l of the recombinant virus stock diluted to 1000-fold was inoculated. After culturing at 37°C in a 5% $CO_2$ incubator overnight, the virus solution was removed and 10 ml of phenol red-free 1% agar CEF growth medium was overlaid. After incubation in an incubator at 37°C in 5% $CO_2$ for about 7 days, plaque formation by the virus in the recombinant virus stock was confirmed. Phenol-red-FCS-free 1% agar CEF growth medium supplemented with 200 $\mu$g/ml CPRG was overlaid and incubation was carried out for 10 hours under the same conditions.

In the plaque derived from the virus stock in which pNZ1127 had been incorporated, red color formation was noted but no such color formation was noted in the plaque derived from the virus stock in which pNZ1127R had been incorporated.

The results reveal that lacZ gene was expressed in pNZ1127 and the nucleotide sequence having the promoter activity was present at the upstream of lacZ gene. On the other hand, lacZ gene was not expressed in pNZ1127R and any nucleotide sequence having the promoter activity was not present at the upstream of lacZ gene.

(6) Shotgun cloning

One microgram each of NP strain DNA obtained in (1) was fully digested with Sau 3AI or Eco RV. The Eco RV digestion product was, after ligating with 10 ng of Bam HI linker (manufactured by Takara Shuzo Co., Ltd.) using T4 DNA ligase, digested again with Bam HI. The product was recovered by 0.8% agarose gel electrophoresis. The thus obtained Sau 3AI fragment and the Eco RV fragment were mixed with 300 ng of pNZ1127R and the mixture was ligated using T4 DNA ligase. This DNA was transformed to Escherichia coli, which had been made competenet by treatment with calcium chloride (hereafter abbreviated as competent Escherichia coli ) [Molecular Cloning, 249-251, Cold Spring Harbour Laboratory, 1982] and cultured in LB agar medium (5 g of NaCl, 5 g of yeast extract, 10 g of Bacto-trypton and 18 g of Bacto-agar per liter) supplemented with 50 μg/ml of ampicillin to give single cell colonies.

(7) Mass production of plasmid pool

Among the colonies obtained in (6), 10 colonies each derived from the Sau 3AI fragment and the Eco RV fragment were charged in 500 ml Sakaguchi flask being filled with 200 ml of sterilized LB liquid medium containing 50 μg/ml of ampicillin (Bacto-agar was withdrawn from LB agar medium). While shaking at 37° C, incubation was performed overnight. Plasmid DNA was prepared from this Escherichia coli according to a modification of the method by Birnboim and Doly [Molecular Cloning (supra), 86-94], which was made plasmid pool. This procedure was repeated to prepare 53 plasmid pools derived from the Sau 3AI fragment and 13 plasmid pools derived from the Eco RV fragment, respectively.

(8) Construction of plasmid pool DNA recombinant virus and plaque assay

Procedures similar to (4) and (5) were carried out except that the plasmid pool DNAs obtained in (7) were used in place of pNZ1127 or pNZ1127R, respectively.
As the result, viruses capable of expressing $\beta$-galactosidase by lacZ gene thereby to decompose CPRG and form a red color were formed. Plasmids transfected to prepare the viruses in the red color-formed plaques were named pNZ1127R/14-6, pNZ1127R/41-3, pNZ1127R/49-2, pNZ1127R/37-1, etc. Among these plasmids, pNZ1127R/14-6, pNZ1127R/41-3 and pNZ1127R/49-2 are derived from the fragment obtained by digesting NP strain DNA with Sau 3AI and, pNZ1127R/37-1 are derived from the fragment obtained by digesting NP strain DNA with Eco RV, in (6).
A size of the plaque formed by these recombinant viruses and their growth property are not different from those of NP strain which is the parent strain, indicating that the Eco RV site into which lacZ has been incorporated is non-essential to infectious growth.
These plasmids contain the nucleotide sequence having the promoter activity of the present invention.

(9) Analysis of sequence

After pNZ131B was digested with Bam HI and Eco RI, a fragment of about 3.1 kbp was isolated by agarose gel electrophoresis. This fragment was mixed with pUC18 digested with Bam HI and Eco RI followed by ligation with T4 DNA ligase. The ligation product was transformed to competent Escherichia coli TGI strain. The transformed TGI was inoculated on LB medium plate containing 50 μg/ml of 5-bromo-4-chloro-3-indolyl-$\beta$-D-galactopyranoside (hereafter abbreviated as X-gal). A white colony which did not express $\beta$-galactosidase was selected. From the colony, DNA was prepared and about 100 bp nucleotide sequence of the about 3.1 kbp fragment at the Bam HI site was determined by the dideoxy method [F. Sanger et al., Proc. Natl. Acad. Sci. USA, 74 , 5463-5467 (1987)] (Fig. 5).
Primer FP-I of 20 nucleotides:
3'-AAGGCTGCCACACGCTCTTA-5'

and primer LP-I of 20 nucleotides:

3'-CCTTGTTGCGTATTGGGACT-5'

were synthesized with DNA synthesizer, Genet AII (manufactured by Nippon Zeon Co., Ltd.), based on the Bam HI-adjacent region at the lacZ site, with reference to the sequence determined as above and to the publication [Shirakawa et al., Gene, 28 , 127-132 (1984)], respectively.

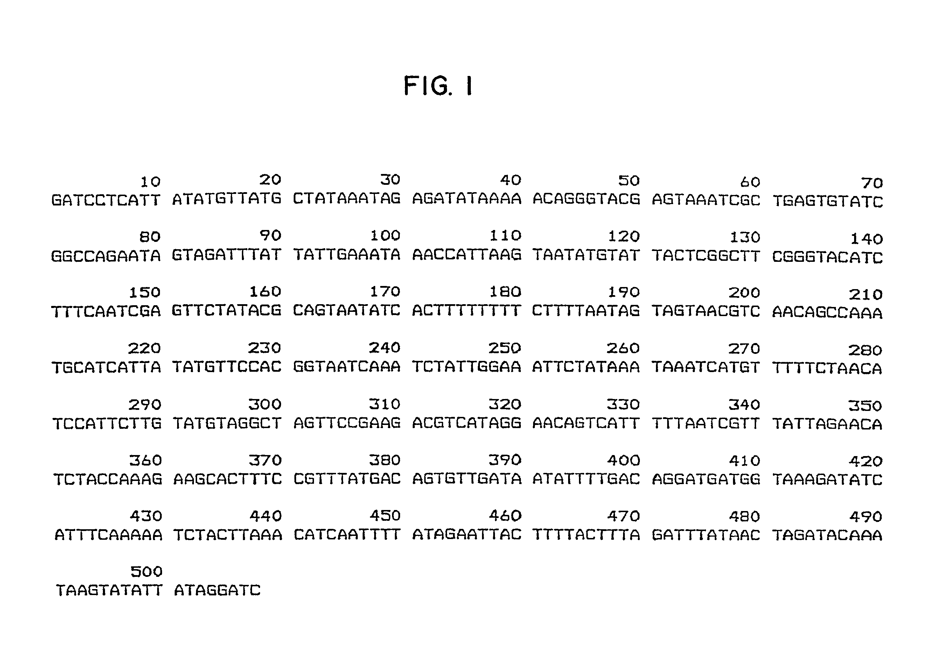

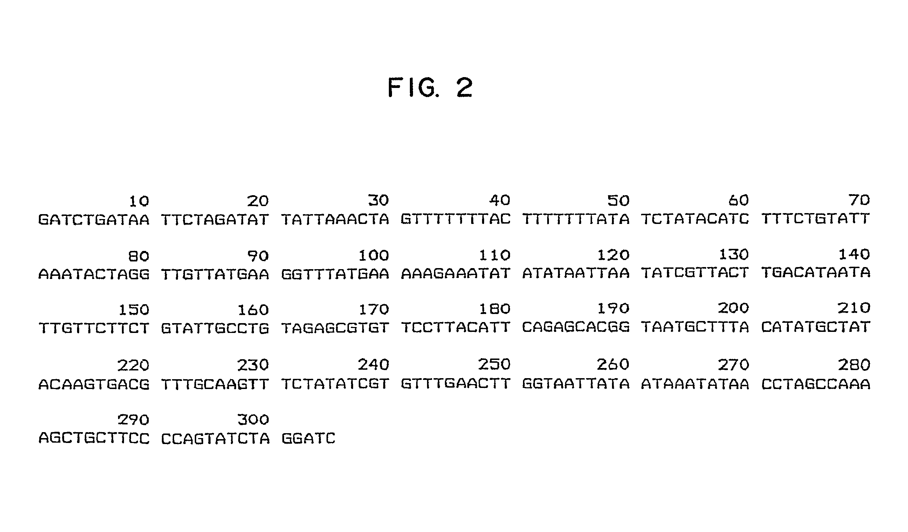

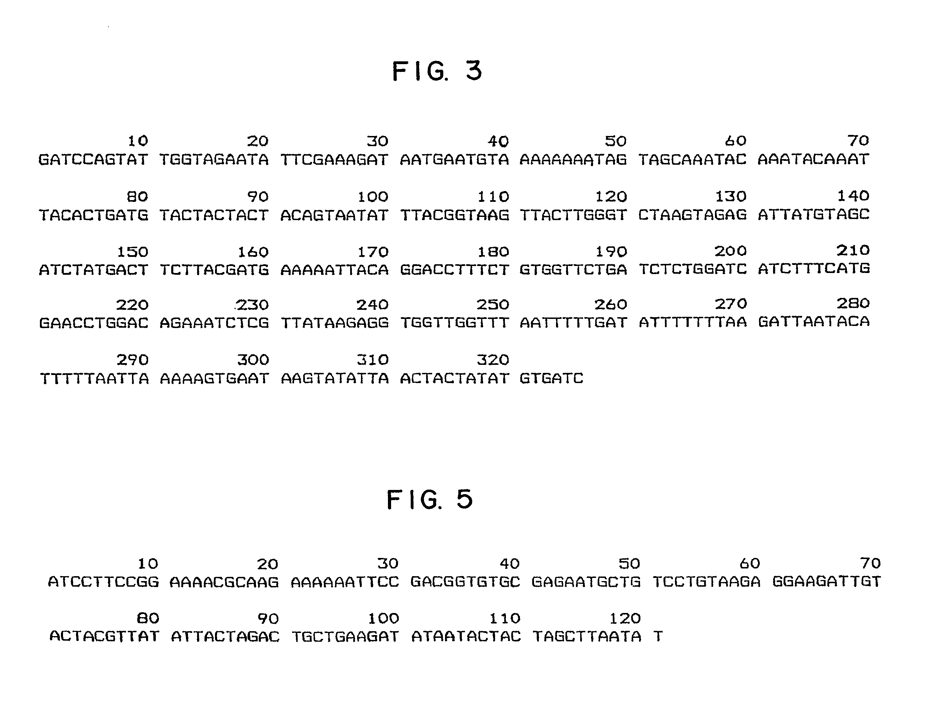

Using these primers, sequences of inserted fragments 14-6, 49-22, 41-3 and 37-1 were directly determined from pNZ1127R/14-6, pNZ1127R/49-22, pNZ1127R/41-3 and pNZ1127R/37-1 by the dideoxy method (which are shown in Figs. 1, 2, 3 and 4, respectively).

These sequences have the promoter activity of the present invention.

Example 2

Construction of recombinant NP strain in which NDV-derived HN gene and NP-derived promoter have been ligated and incorporated so as to express their functions:

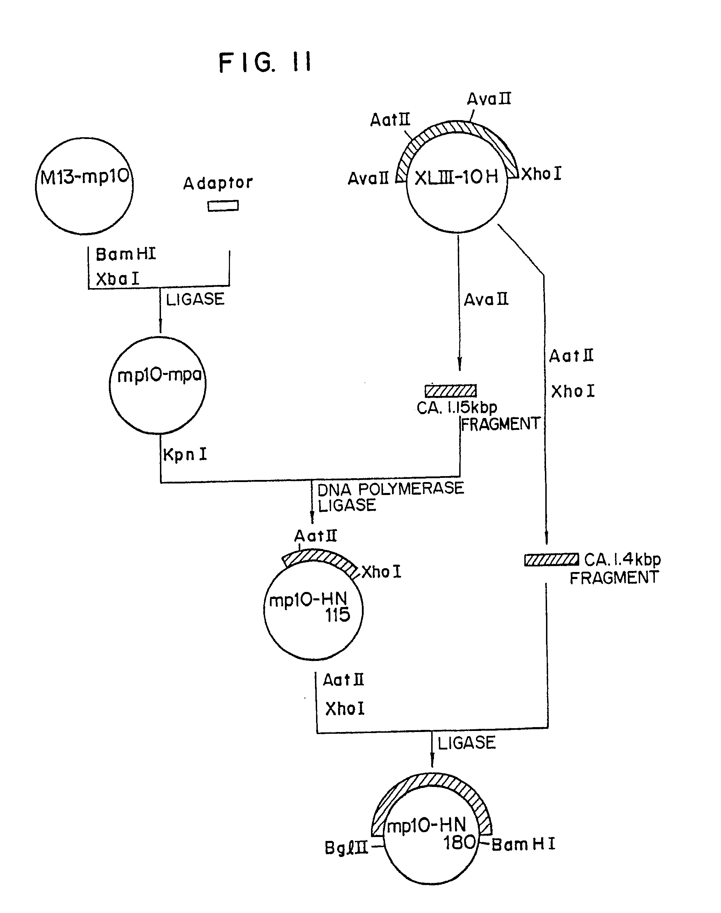

(1) Preparation of hybrid phage mp10-mpa (cf. Fig. 11)

After 0.1 μg of M13-mp10RF DNA (manufactured by Amersham Co.) was digested with Bam HI and Xba I, the digestion product was extracted with phenol-chloroform (1 : 1). By ethanol precipitation, cleaved M13-mp10RF DNA was recovered.

On the other hand, adaptor composed of 40 bp oligodenoxyribonucleotide having the following nucleotide sequence:

CTAGATCTAAGGAGGAAAAAATTATGGTACCTCGAGCTCG

TAGATTCCTCCTTTTTTAATACCATGGAGCTCGAGCCTAG

was chemically synthesized using Genet AIII (manufactured by Nippon Zeon Co., Ltd.). This adaptor was mixed and ligated with the cleaved M13-mp10RF DNA described above using T4 DNA ligase. The product was transformed to competent Escherichia coli TGI strain in a conventional manner [Methods in Enzymoloyg, 101 ]. The transformed TGI strain was cultured at 37°C for 12 hours in 2 x YT agar medium supplemented with 0.003% of X-gal and 0.03 mM isopropyl-β-D-thiogalactopyranoside (hereafter abbreviated as IPTG). Among the formed plaques, phage RFDNA was recovered from a white plaque. The phage was cleaved with Bgl II and a hybrid phage inserted with adaptor DNA was selected by 0.8% agarose gel electrophoresis and named mp10-mpa.

(2) Preparation of hybrid phage mp10-HN115 (cf. Fig. 11)

Plasmid XLIII-10H containing HN gene from NDV [Virus Research, 7 , 241-255 (1987)] was obtained from Assistant Professor Masao Kawakita, of Tokyo University, Department of Liberal Arts. The nucleotide sequence of this plasmid was analyzed by the dideoxy method using M13 phage and, it has been revealed by contrasting to the sequence of known HN gene [J. Gen. Virol., 67 , 1917-1927 (1986)] that cDNA of HN gene is 1746 base pairs from 112 to 1857 shown in Fig. 6. This was clarified by Assistant Professor Kawakita.

The amino acids encoded by the cDNA obtained herein had homology of 95% or more to the amino acid sequence of HN from Beaudette C strain and B₁ strain [E. Jorgensen, Virology, 156 , 12-24 (1987)]. After 4 μg of plasmid XLIII-1 was digested with Ava II, the product was subjected to 1.5% agarose gel electrophoresis. A DNA fragment of about 1.15 kbp was extracted from the agarose gel. By ethanol precipitation, the Ava II fragment of HN gene DNA was recovered.

After hybrid phage mp10-mpa prepared in (1) was digested with Kpn I, extraction was performed with phenol-chloroform (1 : 1). By ethanol precipitation, cleaved mp10-mpa was recovered.

The cleaved mp10-mpa (0.1 μg) was mixed with 0.2 μg of the Ava II fragment (about 1.15 kbp) of the HN gene DNA described above and, the cohesive end of the DNA fragment was rendered blunt end by

8

DNA polymerase. After extracting with phenol-chloroform (1 : 1), the DNA was recovered by ethanol precipitation. The recovered DNA was ligated using T4 DNA ligase and transformed to competent E. coli TGI strain, which was allowed to grow at 37°C for 12 hours in 2 x YT agar medium. Phage RFDNA was recovered from the formed plaque. The phage was cleaved with Xba I and Xho I. A hybrid phage containing the Ava II fragment of HN gene DNA was selected by 0.8% agarose gel electrophoresis and this was named mp10-HN115.

(3) preparation of hybrid phage mp10-HN180 (cf. Fig. 11)

After 4 μg of plasmid XLIII-10H was digested with Aat II and Xho I, the same procedures as in (2) were performed to recover about 1.4 kbp Aat II-Xho I fragment of HN gene DNA.

On the other hand, hybrid phage mp10-HN115 prepared in (2) was digested with Aat II and Xho I. Then, the product was subjected to 0.6% agarose gel electrophoresis to recover about 7.7 kbp Aat II-Xho I fragment.

About 7.7 kbp Aat II-Xho I fragment of HN gene DNA was ligated with about 1.4 kbp Aat II-Xho I fragment of mp10-HN115 by DNA polymerase. The ligation product was transformed to competent E. coli TGI strain, which was allowed to grow at 37°C for 12 hours in 2 x YT agar medium. Phage RFDNA was recovered from the formed plaque. The phage was cleaved with Xho I and Bgl II. A hybrid phage RFDNA containing about 1.8 kbp DNA fragment HN gene was selected by 0.8% agarose gel electrophoresis and this was named mp10-HN180.

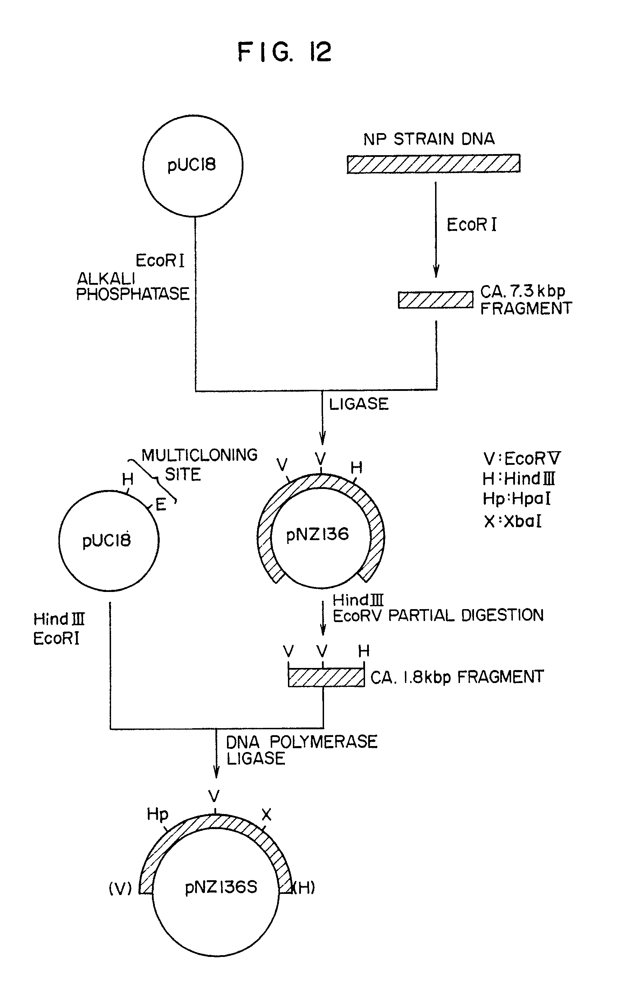

(4) Construction of hybrid plasmid pNZ136S (cf. Fig. 12)

After 2 μg of pUC18 (manufactured by Pharmacia Fine Chemicals, Inc.) was digested with Eco RI and Hind III, extraction was performed with phenol-chloroform (1 : 1). By ethanol precipitation, cleaved pUC18 was recovered. The 5'-end phosphate was removed by treating with alkali phosphatase. After extraction again with phenol-chloroform, DNA was recovered by ethanol precipitation. The cleaved pUC18 (0.2 μg) and 1 μg of the digestion product of the purified NP strain DNA with Eco RI were ligated with each other using T4 DNA ligase. Competent E. coli JM 103 was transformed and cultured at 37°C for 15 hours in LB agar medium supplemented with 0.003% of X-gal, 0.03 mM of IPTG and 40 μg/ml of ampicillin. Among the transformed E. coli grown on the agar medium, white colony was cultured at 37°C for 15 hours in LB liquid medium supplemented with 40 μg/ml of ampicillin and plasmid was extracted by the method of Birnboim and Doly [Nucleic Acid Research, 7 , 1513 (1979)]. After digesting with Eco RI, a hybrid plasmid bearing the fragment having the original NP strain DNA Eco RI fragment (about 7.3 kbp) was detected by 0.6% agarose electrophoresis, which was named pNZ136. A restriction enzyme map of about 7.3 kbp Eco RI fragment is shown in Fig. 6.

After digesting pNZ136 with Hind III, partial digestion was performed with Eco RV. The digestion product was subjected to 0.6% agarose gel electrophoresis to recover about 1.8 kbp Eco RV-Hind III fragment composed of about 0.6 kbp Eco RV-Eco RV fragment and about 1.2 kbp Eco RV-Hind III fragment adjacent to each other.

On the other hand, after pUC18 was digested with Hind III and Eco RI, extraction was performed with phenol-chloroform (1 : 1). By ethanol precipitation, cleaved pUC18 was subjected to 0.8% agarose gel electrophoresis to recover about 2.7 kbp fragment. About 2.7 kbp fragment and about 1.8 kbp fragment, which were rendered the blunt end by DNA polymerase, respectively, were ligated with each other using T4 DNA ligase. Competent E. coli JM 103 was transformed and the transformants were selected in LB agar medium supplemented with ampicillin. A plasmid bearing about 1.8 kbp NP strain DNA fragment was recovered and named pNZ136S.

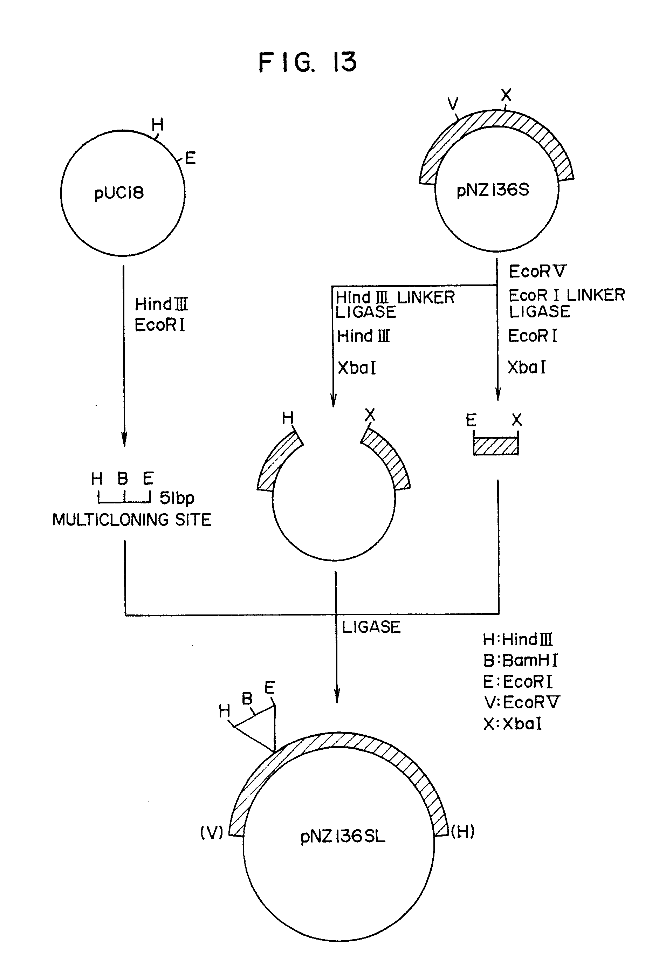

(5) Construction of plasmid pNZ136SL (cf. Fig. 13)

After digesting pNZ136S with Hind III, Eco RI linker (manufactured by Takara Shuzo Co., Ltd.) was ligated therewith using T4 DNA ligase followed by extraction with phenol-chloroform (1 : 1). By ethanol precipitation, DNA was recovered. The recoverd DNA was cleaved with Eco RI and Xba I to recover about 0.2 kbp Eco RI-Xba I fragment. Further after digesting pNZ136S with Eco RV, Hind III linker (manufactured by Takara Shuzo Co., Ltd.) was ligated therewith by T4 DNA ligase followed by extraction with phenol-chloroform (1 : 1). By ethanol precipitation, DNA was recovered. After the recoverd DNA was digested with Hind III and Xba I, about 4.3 kbp Hind III-Xba I fragment was recovered. Further after pUC18 was digested with Hind III and Eco RI, 51 bp fragment at the polyliker site was recovered by 2.0% agarose gel electrophoresis. The thus obtained about 0.2 kbp fragment, about 4.3 kbp fragment and 51 bp fragment were ligated by ligase and a plasmid ligated with the three fragments was extracted, which was named pNZ136SL.

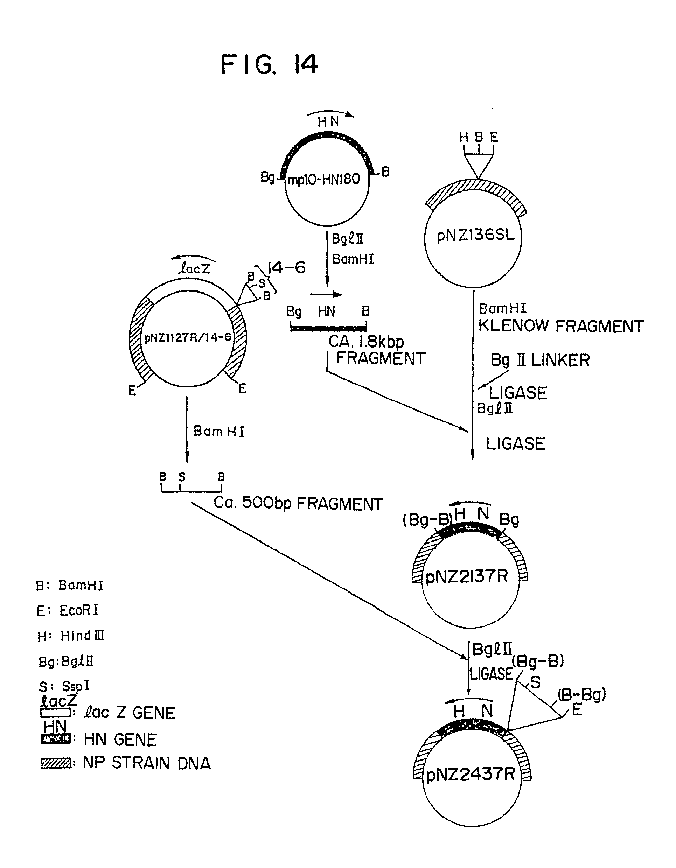

(6) Construction of plasmid pNZ2437R (cf. Fig. 14)

After 10 mg of mp10-HN180 constructed in (3) was fully digested with Bam HI and Bgl II, 0.8% agarose gel electrophoresis was performed to recover HN gene from about 1.8 kbp band.

After 1 $\mu$g of pNZ136SL was digested with Bam HI, it was made blunt end by Klenow fragment. Then 0.1 $\mu$g of Bgl II linker was added thereto followed by ligation using T4 DNA ligase. After digesting with Bgl II, about 0.5 $\mu$g of the HN gene fragment described above was added thereto followed by ligation using T4 DNA ligase. Then, a plasmid in which one copy of the HN gene had been inserted as shown in Fig. l4 was selected and named pNZ2137R.

Clone 14-6 fragment cloned to 10 $\mu$g of promoter vector pNZ1127R was excised with Bam HI and recovered.

After 1 $\mu$g of pNZ2137R was digested with Bgl II, 0.5 $\mu$g of the excised clone 14-6 fragment was added thereto followed by ligation using T4 DNA ligase. Among them, a plasmid in which one copy of 14-6 had been inserted was selected in terms of its molecular weight and the promoter had been ligated with HN gene in such a direction as to be capable of reaction through digestion with Ssp I and Eco RI was selected. This was made recombinant plasmid pNZ2437R.

(7) Construction of recombinant NP strain using pNZ2437R:

Recombinant virus stock containing recombinant NP strain bearing HN gene thereon was obtained in a manner similar to Example 1 (4) except for using pNZ2437R in place of pNZ1127 or pNZ1127R.

(8) Plaque hybridization and selection of recombinant NP strain:

A 10-fold dilution (500 $\mu$l) of the recombinant virus stock was inoculated on 1.0 x 10$^7$ CEF cultured at 37$^\circ$C in a 10 cm Petri dish in 5% CO$_2$ incubator. The inoculant was again put in 5% CO$_2$ incubator at 37$^\circ$C. Two hours after, the virus solution was removed and CEF was then washed twice with Eagle's MEM. CEF growth medium containing 1% agar was overlaid in a volume of 10 ml. After incubation at 37$^\circ$C in 5% CO$_2$ incubator for about 7 days, plaque appeared. After the plaque formation was confirmed, 10 ml of FCS-free CEF growth medium containing 0.02% neutral red and 1% agar was overlaid. After incubation at 37$^\circ$C for 4 to 5 hours, a cell group which was not stained red was confirmed and checked on a plate. The overlaid agar medium was transferred to another dish so as not to destroy its shape.

A nylone membrane (manufactured by Pole Co.) was pushed onto the cell layer remained at the bottom of the Petri dish to transfer virus thereon. After air drying, the membrane was treated with a modifier (0.5 M NaOH and 1.5 M NaCl) for 10 minutes and then with a neutralizing solution (3 M sodium acetate, pH 5.5) for 10 minutes. After air drying, the membrane was heated at 80$^\circ$C for 2 hours. The membrane was treated with 4 x STE (0.6 M NaCl, 0.08 M Tris HCl, 4 mM EDTA, pH 7.8)-10 x Denhardt (1% polyvinylpyrrolidone, 1% Ficoll)-0.1% SDS at 68$^\circ$C for 2 hours. 4 x STE-10 x Denhardt-0.1% SDS-0.1% Na$_4$P$_2$O$_7$-50 $\mu$g/ml-modified salmon sperm DNA and cDNA coding for labeled NDV HN were hybridized by nick translation at 68$^\circ$C for 14 hours. After washing, the nylon membrane was put on an X ray film, which was subjected to autoradiography to confirm the presence of a spot. The X ray film was overlaid on the agar kept at 4$^\circ$C and a plaque coincided with the spot was identified to be a recombinant containing HN gene. The recombinant was isolated with a sterilized Pasteur pipette. The obtained recombinant virus was named fNZ2437R.

(9) Purification of recombinant NP strain containing HN gene of NDV

The recombinant was purified by repeating the procedures similar to those in (8) except that 200 $\mu$l of a

suspension of plaque containing fNZ2437R isolated in 1 ml of Eagle's MEM was used in place of 500 $\mu$l of the 10-fold dilution of recombinant virus stock.

With respect to fNZ2437R, all plaques became spots on an X ray film by repeating the procedures 4 times, indicating that purification was completed.

(10) Expression of HN gene in cells by the recombinant NP strain (fluorescent antibody technique)

The aforesaid recombinant virus fNZ2437R showing m.o.i of 1.0 was inoculated on CEF cultured in a chamber slide for tissue culture. After culturing at 37°C in a 5% $CO_2$ incubator, FCS-free CEF growth medium was added. Thereafter, incubation was performed for 2 days at 37°C in a 5% $CO_2$ incubator and the medium was then withdrawn. After washing with Dulbecco-PBS (-) and air drying, the system was treated with cold acetone for 20 minutes to fix the cells. By examining in accordance with immunofluorescent technique using as a first antibody anti-NDV chick antibody and as a second antibody fluorescein isothiocyanate-bound anti-chick IgG antibody, it was confirmed that the recombinant NP strain had HN protein productivity of NDV.

(11) Expression of HN gene in cells by the recombinant NP strain (enzyme antibody technique)

The recombinant virus solution (500 $\mu$l) obtained in (9) was inoculated on 1 x $10^7$ CEF cultured in a 10 cm Petri dish in a concentration of 1 x $10^2$ PUF/ml. Two hours after, 10 ml of CEF growth medium containing 1% agar was overlaid followed by incubation at 37°C in 5% $CO_2$ incubator for 7 days to form plaques.

The agar medium was withdrawn from the Petri dish. A sterilized nylon membrane was pushed onto the surface of cells remained at the bottom of the Petri dish to transfer virus plaques and cells thereon. After treating with 2% skimmed milk-supplemented Dulbecco-PBS (-) for 30 minutes, anti-NDV chicken antibody was reacted as a first antibody at room temperature for an hour. After the reaction, the mixture was washed with Dulbecco-PBS (-) and biotinated anti-chicken IgG antibody was reacted as a second antibody at room temperature for an hour. After the reaction, the mixture was washed with Dulbecco-PBS (-) and horse radish peroxidase-bound avidin-biotin complex was reacted at room temperature for 30 minutes. After the reaction, it was colored with 8 mM Tris-hydrochloride buffer (pH 8.0) containing 0.05% 4-chloro-l-naphthol and 0.3% $H_2O_2$. By checking with the foregoing enzyme antibody method, it was confirmed that the recombinant NP strains had NDV HN protein productivity.

Example 3

Construction of recombinant NP strain capable of concurrently expressing surface antigen HN of Newcastle disease virus and lacZ gene of Escherichia coli under the control of different NP strain promoters:

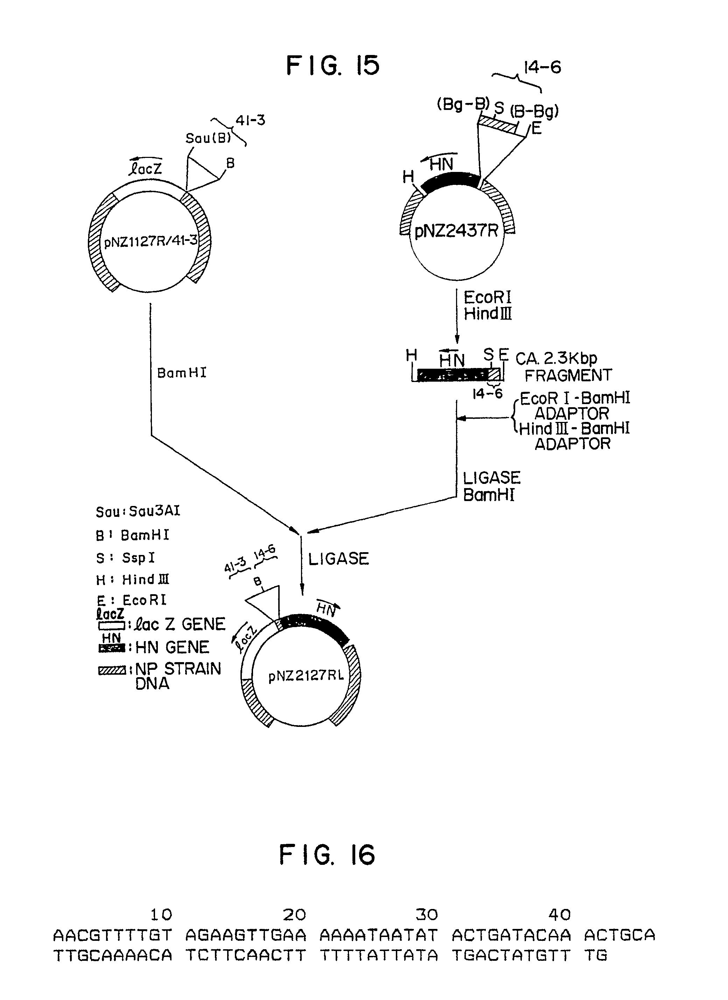

(1) Construction of recombinant plasmid pNZ2127RL (Fig. 15)

After 10 $\mu$l of plasmid pNZ2437R constructed in Example 2 (6) was digested with Eco RI and Hind III, 0.8% agarose gel electrophoresis was performed to recover a band of about 2.3 kbp. To 3 $\mu$g of the DNA fragment out of the band were added both 0.1 $\mu$g of Eco RI-Bam HI adaptor and 0.1 $\mu$g of Hind III-Bam HI adaptor followed by ligation using T4 DNA ligase. After digesting the ligation product with Bam HI, 0.8% agarose gel electrophoresis was again performed to recover a band of about 2.3 kbp.

After 0.1 $\mu$g of recombinant plasmid pNZ1127R/41-3 in which the 41-3 fragment had been ligated before lacZ was digested with Bam HI, 1 $\mu$g of the fragment of about 2.3 kbp recovered from pNZ2437R described above was added to the digestion product followed by ligation using T4 DNA ligase. After digestion with Eco RI, a plasmid shown in Fig. 14 was selected from the 0.8% agarose gel electrophoresis pattern and named pNZ2127RL.

(2) Construction of recombinant NP strain using pNZ2127RL

A reccmbinant virus stock containing recombinant NP strain bearing HN gene and lacZ gene was obtained in a manner similar to Example 1 (4) except for using pNZ2127RL in place of pNZ1127 or pNZ1127R.

### (3) Selection and purification of recombinant NP strain

Using the recombinant virus stock obtained in (2) above, the same procedures as in Example 1 (5) were carried out. A plaque which formed a red color was recovered through a sterilized Pasteur pipette and suspended in 1 ml of Eagle's MEM to prepare a recombinant virus stock. Purification was performed by repeating the procedures similar to Example (5) until all plaques appeared formed a red color.

The same procedures as in Example 2 (8) were carried out except that 200 $\mu$l of a suspension of one of the purified plaques recovered through a Pasteur pipette in 1 ml of Eagle's MEM was used in place of 500 $\mu$l of the recombinant virus stock. As the result, it was confirmed that HN gene had been incorporated in the virus of all plaques.

With respect to the purified virus, plaque hybridization was performed in a manner similar to Example 2 (8). In all plaques produced by the purified virus, spots were noted on an X ray film, indicating that HN gene had also been incorporated.

### (4) Expression of HN gene in cells by the recombinant NP strain (fluorescent antibody technique, enzyme antibody technique)

Expression of HN protein by the recombinant NP strain was confirmed in a manner similar to Example 2 (10) and (11) except for using the recombinant virus obtained in (3) instead of fNZ2437.

### Example 4 Collection of a moiety necessary for the promoter activity of DNA fragment 37-1

### (1) Plasmid library of NP strain genome DNA and Southern blotting hybridization

Using T4 DNA ligase, the digestion product of NP strain DNA extracted in Example 1 (1) with Eco RI was ligated with the digestion product of pUC18 with Eco RI and the resulting ligation product was transformed to competent Escherichia coli TGI strain [Molecular Cloning, 249-251, Cold Spring Harbour Laboratory, 1982]. The transformant was cultured in LB agar medium supplemented with 0.8 $\mu$l/ml of X-gal and 50 $\mu$g/ml of ampicillin and, a white single cell colony was selected. The colony was inoculated on 200 ml of sterilized LB liquid medium supplemented with 50 $\mu$g/ml of ampicillin which was charged in a 500 ml Sakaguchi flask. It was incubated with shaking at 37°C overnight. Plasmid DNA was prepared from these Escherichia coli according to a modification of the method by Birnboim and Doly (supra) and, a restriction enzyme map of the inserted fragment was prepared using Eco RI, Hind III and Bam HI. Comparison with information on known APV genome (C.C. Randall et al., Virology, 89 , 229-239 (1978), etc.) shows that the Eco RI fragment of the cloned NP strain DNA had about 80% of the nucleotide sequence of NP strain genome.

After pNZ1127R/37-1 was digested with Bam HI, electrophoresis was carried out on 1.5% agarose gel to prepare the isolated DNA fragment of about 600 bp. Using the DNA fragment as template DNA, [32]p-labeled probe DNA was prepared according to multi-prime DNA labeling system (manufactured by Amersham Co.). After the aforesaid plasmid DNA having about 80% of the nucleotide sequence of NP strain genome was digested with Eco RI, 0.8% agarose gel electrophoresis was performed. Using this probe DNA, Southern blotting hybridization and autoradiography were conducted. The results revealed that the nucleotide sequence having homology to the probe DNA was present in the NP strain-derived DNA of about 8 kbp cloned to pNZ136.

After pNZ136 was digested with Eco RI and Hind III, electrophoresis was carried out on 0.5% agarose gel. Using the aforesaid probe DNA, Sounthern plotting hybridization and autoradiography were conducted. The results revealed that the nucleotide sequence having homology to the probe DNA was present in the Hind III fragment of about 4 kbp excised from the NP strain-derived DNA of about 8 kbp described above.

(2) Cloning of the Hind III fragment of about 4 kbp in M13 phage DNA and deletion of DNA

After pNZ136 was digested with Hind III, 0.8% agarose gel electrophoresis was conducted to recover the DNA fragment of about 4 kbp. This fragment was ligated with M13 mp19RF DNA at the Hind III site using T4 DNA ligase, which was then transfected to competent Escherichia coli TGI strain. This Escherichia coli was mixed with LB soft agar medium (in LB agar medium, the amount of Bacto-agar was changed to 8 g) containing 0.003% of X-gal and 0.03 mM of IPTG, prior to solidification. The resulting mixture was overlaid on LB agar medium followed by incubation at 37°C overnight. Twelve plaques appeared which did not take on blue color were collected.

Each of the plaques was inoculated on 200 ml of sterilized LB liquid medium supplemented with 50 µg/ml of ampicillin which was charged in a 500 ml Sakaguchi flask. While shaking at 37°C, incubation was performed overnight to prepare RF DNAs of M13.

After these RF DNAs were digested with Hpa I and Pst I, 0.8% agarose gel electrophoresis was conducted and was selected RF DNAs, one by one, in which the Hind III fragment of about 4 kbp had been cloned to directions different from each other, was selected from the pattern. The two M13 RF DNAs were digested with Kpn I and Sam HI and then treated with Kilosequence Deletion Kit (manufactured by Takara Shuzo Co., Ltd.). The treated matter was transfected to competent Escherichia coli TGI strain and mixed with LB soft agar medium before solidification. The resulting mixture was overlaid on LB agar medium followed by incubation at 37°C overnight. The plaques appeared were collected.

Each of the plaques was inoculated on 200 ml of sterilized LB liquid medium which was charged in a 500 ml Sakaguchi flask. While shaking at 37°C, incubation was performed overnight to prepare RF DNAs of M13.

After these RF DNAs were digested with Hind III and Eco RI, 0.8% agarose gel electrophoresis was conducted. From the Hind III fragment of about 4 kbp inserted, M13 RF DNAs of about 3.7 kbp, about 3.4 kbp and about 3.1 kbp, of which deletion was noted by about 300 bp each, were selected.

(3) Sequence analysis of the Hind III fragment of about 4 kbp derived from NP strain DNA

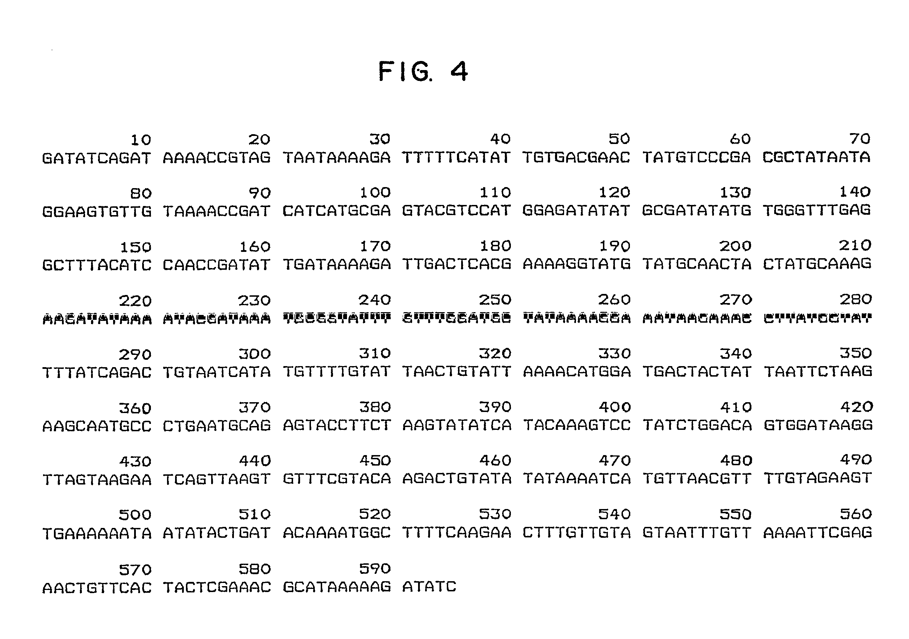

Various M13 RF DNAs prepared in (2) were transfected to competent Escherichia coli TGI strain. After shake culture in 2 ml of LB liquid medium for 8 hours, the medium was transferred to a micro centrifuging tube and centrifuged at 15,000 rpm for 30 seconds in a cooling centrifuge (MR15A, manufactured by Tomy Seiko Co., Ltd.). From the medium, 1 ml of the supernatant was collected. After 200 µl of PEG/NaCl aqueous solution (20% polyethylene glycol 6000, 2.5 M sodium chloride) was added to the supernatant, the mixture was again centrifuged at 15,000 rpm for 5 minutes in a cooling centrifuge. Recombinant M13 phage obtained as the residue was extracted with 50 µl of TE and 50 µl of phenol/chloroform (1 : 1) and the extract was precipitated with ethanol. The thus recovered DNA was washed with 70% ethanol and air-dried to prepare single-stranded DNA. Using M2 primer (manufactured by Takara Shuzo Co., Ltd.), the sequence of each single-stranded DNA was determined by the dideoxy method. The results revealed that the entire nucleotide sequence of the DNA fragment 37-1 was a part of the nucleotide sequence of this Hind III fragment of about 4 kbp. The results also revealed that an open reading frame composed of 708 nucleotides following initiation codon starting with the 516th nucleotide in Fig. 4 was present.

(4) Preparation of DNA fragments △14, △26 and △36

The results of sequence analysis revealed that plasmid pNZ136SL was a plasmid in which NP strain DNA of about 1.8 kbp containing the entire nucleotide sequence following the 4th nucleotide toward the downstream of the DNA fragment 37-1 shown in Fig. 4 was cloned and multicloning site of pUC18 was further incorporated into the Eco RV site starting with 590th nucleotide.

The synthetic DNA having a sequence shown in Fig. 16 was ligated with the DNA fragment of 4.5 kbp recovered from the digestion product of pNZ136SL with Hpa I and Pst I by 0.8% agarose gel electrophoresis, using T4 DNA ligase and the resulting plasmid was named pNZ1365P.

pNZ136SP was digested with Nco I and treated with Kilosequence Delation Kit. The thus treated plasmid was ligated with synthetic DNA:

5'-AAGCTTAGATCTAAGCTT-3'

3'-TTCGAATCTAGATTCGAA-5'

using T4 DNA ligase. The thus obtained plasmid was transfected to competent Escherichia coli TGI strain. Colonies were formed on LB agar medium supplemented with 50 µg/ml of ampicillin.

Plasmid DNA was prepared from each colony in a manner similar to Example 1 (7) except that 10 colonies obtained were independently used instead of combining 10 each of the two colonies.

From the plasmid DNAs, DNA not cleaved with Nco I was selected and digested with Bam HI and Bgl II followed by 6% polyacrylamide gel electrophoresis. The DNA fragment of about 300 bp, the DNA fragment of about 200 bp and the DNA fragment of about 100 bp were recovered and named △14, △26 and △36, respectively.

(5) Preparation of DNA fragment △N

pNZ136SP was digested with Nco I and was treated with Klenow fragment. The thus treated plasmid was ligated with synthetic DNA:

5'-AAGCTTAGATCTAAGCTT-3'

3'-TTCGAATCTAGATTCGAA-5'

using T4 DNA ligase. The thus obtained plasmid was transfected to competent Escherichia coli TGI strain. Colonies were formed on LB agar medium supplemented with 50 µg/ml of ampicillin. Plasmid DNA was prepared from each colony in a manner similar to Example 1 (7) except that 10 colonies obtained were independently used instead of combining 10 each of the two colonies.

From the thus prepared plasmid DNAs, DNA cleaved with Hind III was selected and digested with Bam HI and Bgl II. The DNA fragment of about 300 bp was recovered and named △ N.

(6) Preparation of DNA fragment △Hpa I

The DNA fragment of about 50 bp was recovered in a manner similar to (5) except for using Hpa I instead of Nco I. The DNA fragment was named △Hpa I.

(7) Sequence analysis of DNA fragment

Each of the five DNA fragments obtained in (4), (5) and (6) was liaged with pNZ1127R digested with Bam HI, using T4 DNA ligase. The thus obtained plasmid was transfected to competent Escherichia coli TGI strain. Colonies were formed on LB agar medium supplemented with 50 µg/ml of ampicillin. Plasmid DNAs were prepared from each colony in a manner similar to Example 1 (7) except that 10 colonies (50 in total) obtained with each of the DNA fragments were independently used one by one, instead of combining 10 each for the two colonies.

With respect to the resulting plasmids, their sequences were determined by the dideoxy method, using primers FP-1 and LP-1. The results revealed that △14, △26, △36, △N and △Hpa I have nucleotide sequences of 225 to 515 nucleotides, 301 to 515 nucleotides, 404 to 515 nucleotides, 3 to 515 nucleotides, 475 to 515 nucleotides, respectively, shown in Fig. 4.

Furthermore, plasmids in which the five DNA fragments had been inserted by one fragment each in the same direction as that of the same nucleotide sequence in pNZ1127R/37-1 were selected and named pNZ1127R/△14, pNZ1127R/△26, pNZ1127R/△36, pNZ1127R/△N and pNZ1127R/△Hpa I, respectively.

(8) Determination of promoter activity in DNA fragment 37-1-derived DNA fragment

Recombinant virus stock was obtained in a manner similar to Example 1 (4) except for using the five plasmids obtained in (7) instead of pNZ1127 and pNZ1127R.

This recombinant virus stock was subjected to plaque assay in a manner similar to Example 1 (5). In any cells infected with the recombinant virus, red plaques were noted and it was revealed that all of the DNA fragments △14, △26, △36, △N and △Hpa I had the promoter activity.

Red plaques were collected. Using the red plaque as a virus stock, the recombinant virus was purified until all plaques appeared to form a red color.

The recombinant viruses obtained from pNZ1127R/△14, pNZ1127R/△26, pNZ1127R/△36, pNZ1127R/△N and pNZ1127R/△Hpa I were named fNZ1127R/△14, fNZ1127R/△26, fNZ1127R/△36, fNZ1127R/△N and fNZ1127R/△Hpa I, respectively.

Each of the purified viruses were cultured overnight and inoculated on CEF cultured in a multiplicity of $6 \times 10^5$ PFU to infect $3 \times 10^6$ of CEF. After culturing at 37°C in 5% $CO_2$ incubator for 15 hours, the viruses were frozen and thawed 3 times. Using the viruses, their $\beta$-galactosidase activity was determined according to the method of Miller [J.H. Miller, Experiment of Molecular Genetics, 352-355, Cold Spring Harbour Laboratory (1972)]. The results are shown in Table 1.

### Table 1

| Virus | Relative Activity |
|-------|-------------------|
| fNZ1127R/37-1 | 1.0 |
| fNZ1127R/△N | 1.1 |
| fNZ1127R/△14 | 1.2 |
| fNZ1127R/△26 | 1.1 |
| fNZ1127R/△36 | 1.2 |
| fNZ1127R/△Hpa I | 0.1 |

The relative activity is expressed by a relative value to $\beta$-galactosidase activity of fNZ1127R/37-1.

Example 5 Examination of promoter capable of expressing marker gene by DNA fragment 37-1-derived DNA fragment

(1) Examination of promoter by DNA fragment 37-1-derived synthetic DNA fragment

PS-17 containing nucleotide sequence from 489 to 505 nucleotides shown in Fig. 4:

```
5'-GATCGTTGAAAAAATAATATA     -3'

3'-     CAACTTTTTTATTATATCTAG-5'
```

and PS-9 containing nucleotide sequence from 497 to 505 nucleotides shown in Fig. 4:

```
5'-GATCAATAATATA     -3'

3'-     TTATTATATCTAG-5'
```

were synthesized.

pNZ1127R was digested with Bam HI and PS-17 and PS-9 were added thereto, respectively, to ligate them using T4 DNA ligase. The thus obtained plasmids were transfected to competent Escherichia coli TG1 strain. Colonies were formed on LB agar medium supplemented with 50 μg/ml of ampicillin. Plasmid DNA was prepared from each colony in a manner similar to Example 1 (7) except that the colonies obtained were independently used instead of combining 10 each of the two colonies.

From the plasmid DNAs, DNA not cleaved with Bam HI was selected. By sequence analysis using primers LP-1 and FP-1 according to the dideoxy method, plasmids in which one fragment each had been inserted in the same direction as that of the same nucleotide sequence in pNZ1127R/37-1 were selected.

Recombinant stock virus was obtained in a manner similar to Example 1 (4) except for using the two plasmids in place of pNZ1127 or pNZ1127R, respectively.

This recombinant virus stock was subjected to plaque assay in a manner similar to Example 1 (5). Only the plaque formed by the recombinant virus containing the nucleotide sequence of PS-17 at the upstream of lacZ gene was stained red. The results revealed that PS-17 had the promoter activity.

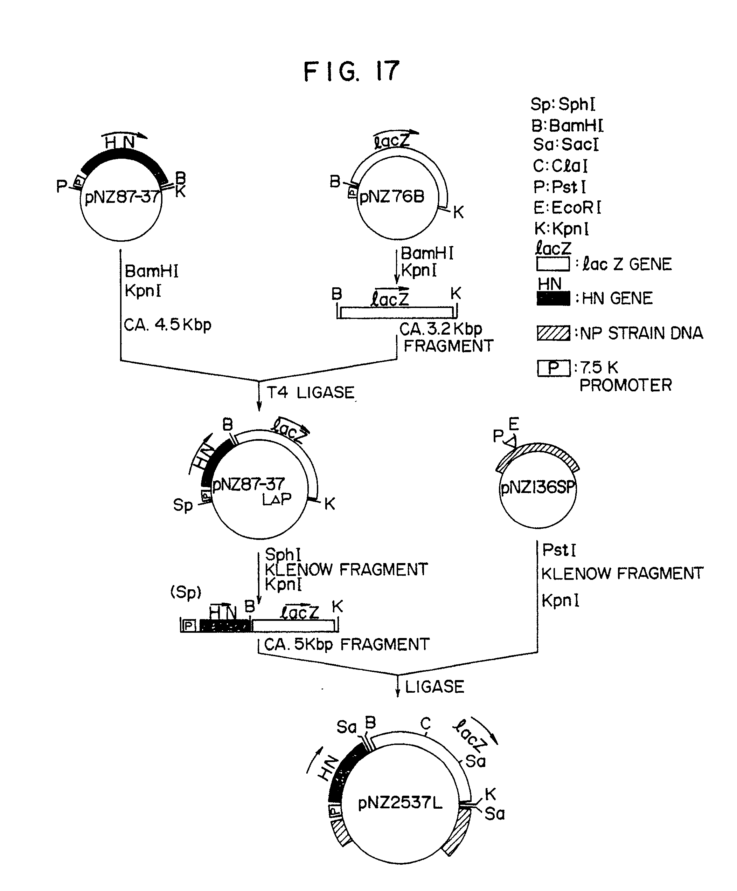

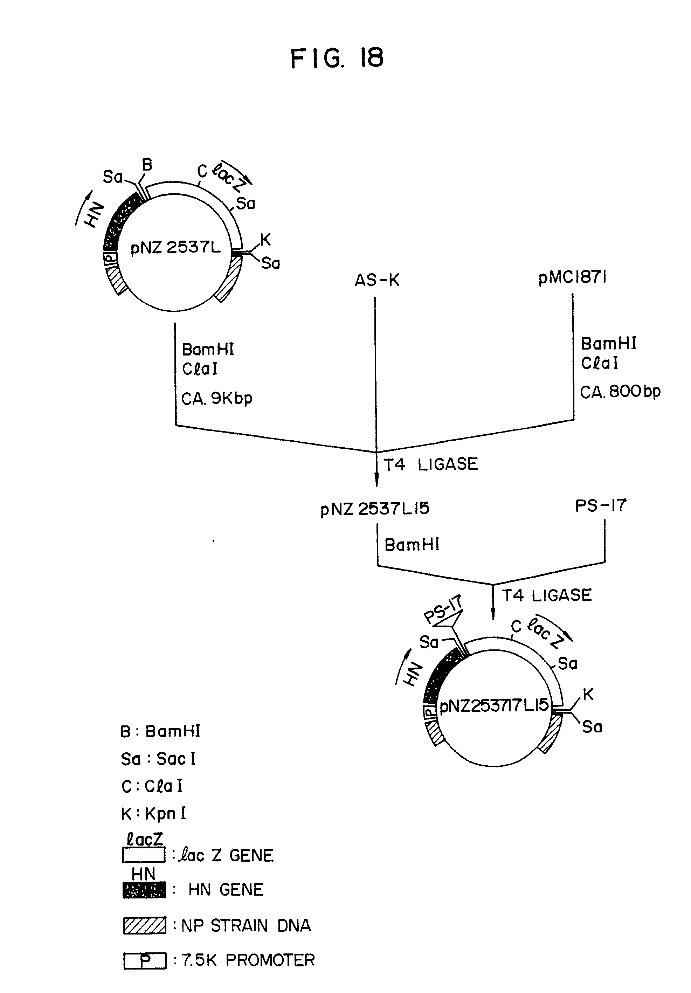

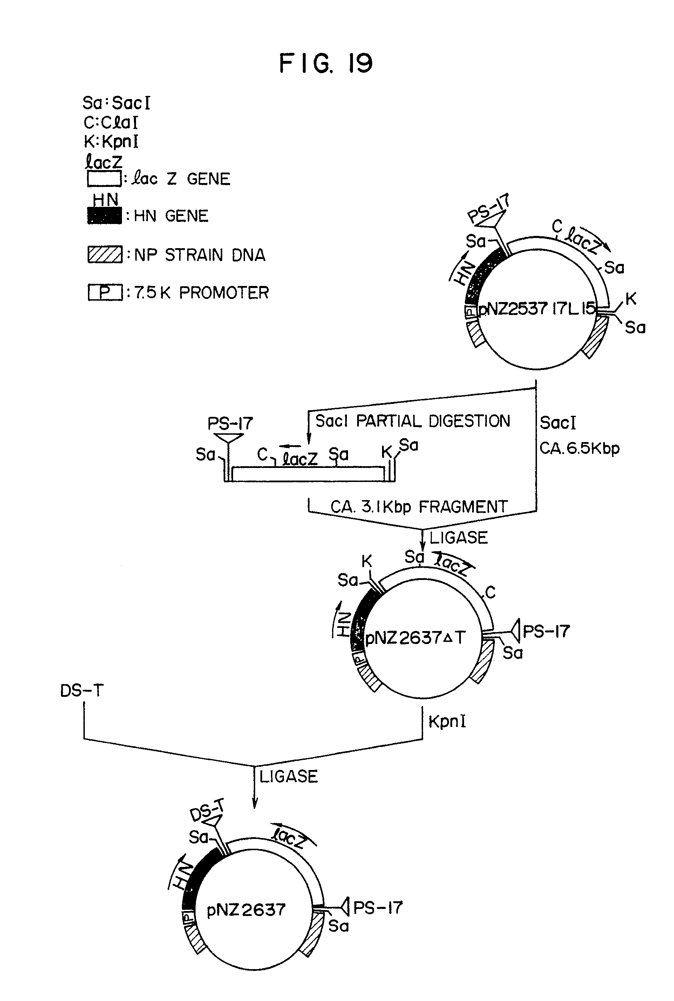

### (2) Construction-of plasmid pNZ2637 (cf. Figs. 17, 18 and 19)

After pNZ87-37 (Japanese Patent Application Laid-Open No. 1-157381), in which DNA fragment having ligated HN gene of NDV D-26 strain under control of 7.5 K promoter of vaccinia virus had been cloned to pUC18, was digested with Bam HI and Kpn I, 0.8% agarose gel electrophoresis was performed to recover DNA fragment of about 4.5 kbp. Separately, pNZ76B was digested with Bam HI and Kpn I and 0.8% agarose gel electrophoresis was then performed to recover DNA fragment of about 3.2 kbp containing lacZ gene. The two DNA fragments were ligated with each other using T4 DNA ligase to construct pNZ87-37LΔP.

After pNZ87-37LΔP was digested with Sph I and then treated with Klenow fragment, the treated matter was digested with Kpn I and 0.8% agarose gel electrophoresis was performed to recover DNA fragment of about 5 kbp. Furthermore, pNZ136SP was digested with Sph I and treated with Klenow fragment followed by digestion with Kpn I. The resulting digestion product was ligated with the aforesaid DNA fragment of about 5 kbp using T4 DNA ligase to construct pNZ2537L.

After pMC1871 (manufactured by Pharmacia Fine Chemicals, Inc.) was digested with Bam HI and Cla I, 0.8% agarose gel electrophoresis was performed to recover DNA fragment of about 800 bp. Further after pN2537L was digested with Bam HI and Cla I, 0.8% agarose gel electrophoresis was performed to recover DNA fragment of about 9 kbp. These two DNA fragments were ligated with synthetic DNA AS-K:

$$5'-GATCCTAAAATGGAA \qquad -3'$$

$$3'- \qquad GATTTTACCTTCTAG-5'$$

using T4 DNA ligase. The thus obtained plasmid was transfected to competent Escherichia coli TGI strain. Colonies were formed on LB agar medium supplemented with 50 μg/ml of ampicillin. Plasmid DNA was prepared from each colony in a manner similar to Example 1 (7) except that the colonies obtained were independently used instead of combining 10 each of the two colonies.

From the plasmid DNAs, DNA in which AS-K had been inserted between lacZ gene and HN gene and nucleotide sequence STG in AS-K was ligated to act as initiation codon of lacZ gene was selected by sequence analysis, which was named pNZ2537L15.

pNZ2537L15 was digested with Bam HI followed by ligation with PS-17 using T4 DNA ligase. The thus obtained plasmid was transfected to competent Escherichia coli TGI strain. Colonies were formed on LB agar medium supplemented with 50 μg/ml of ampicillin. Plasmid DNA was prepared from each colony in a manner similar to Example 1 (7) except that 10 colonies obtained were independently used instead of combining 10 each of the two colonies. From the plasmid DNAs, DNA which was not cleaved with Bam HI and in which only one fragment of PS-17 was inserted in the direction considered to govern lacZ gene as promoter was selected. This plasmid DNA was named pNZ253717L15.

After pNZ253717L15 was partially digested with Sac I, 0.8% agarose gel electrophoresis was performed to recover DNA fragment of about 3.2 kbp containing pS-17 and the entire length of lacZ gene. Further after pNZ253717L15 was fully digested with Sac I, 0.8% agarose gel electrophoresis was performed to recover DNA fragment of about 6.5 kbp. These two DNA fragments were ligated with each other using T4 DNA ligase. The thus obtained plasmid was transfected to competent Escherichia coli TGI strain. Colonies were

formed on LB agar medium supplemented with 50 μg/ml of ampicillin. Plasmid DNA was prepared from each colony in a manner similar to Example 1 (7) except that the colonies obtained were independently used instead of combining 10 each of the two colonies. After this plasmid was digested with Cla I and Pst I, 0.8% agarose gel electrophoresis. Based on its pattern, plasmid in which only one fragment of lacZ gene had been inserted in the direction opposite that of pNZ253717L15 was selected and named pNZ2637△T.

Recombinant virus stock was obtained in a manner similar to Example 1 (4) except for using pNZ2637△T instead of pNZ1127 and pNZ1127R.

This recombinant virus stock was subjected to plaque assay in a manner similar to Example 1 (5). Red-stained plaques were noted.

Red plaques were collected. Using the red plaque as a virus stock, the recombinant virus was purified until all plaques appeared were stained red.

The thus obtained recombinant virus which expressed β-galactosidase was named fNZ2637△T.

After cleaving between HN gene and lacZ gene of fNZ2637△T with Kpn I, the cleavage product was ligated with synthetic DNA DS-T:

$$5'- \quad \text{ATTTTTATAAAAATCTAG}-3'$$

$$3'-\text{GATCTAAAAATATTTTTA} \quad -5'$$

using T4 DNA ligase to insert them. The thus obtained plasmid was transfected to competent Escherichia coli TGI strain. Colonies were formed on LB agar medium supplemented with 50 μg/ml of ampicillin. Plasmid DNA was prepared from each colony in a manner similar to Example 1 (7) except that 10 colonies obtained were independently used instead of combining 10 each of the two colonies. The thus obtained plasmid DNAs were analyzed with their sequences and plasmid in which only one fragment of DS-T had been inserted at the Kpn I site was selected and named pNZ2637.

(3) Expression of lacZ gene by fNZ2637 and its stability

Recombinant virus stock was obtained in a manner similar to Example 1 (4) except for using pNZ2637 instead of pNZ1127 and pNZ1127R.

This recombinant virus stock was subjected to plaque assay in a manner similar to Example 1 (5). Red-stained plaques were noted.

Red plaques were collected. Using the red plaque as a virus stock, the recombinant virus was purified until all plaques appeared were stained red.

The thus obtained recombinant virus which expressed β-galactosidase was named fNZ2637.

After $1.0 \times 10^6$ of CEF cultured in 25 cm² culture bottle was infected with fNZ2637, incubation was carried out at 37°C in a 5% $CO_2$ incubator for 3 days. The virus was frozen and thawed 3 times and again infected to $5 \times 10^6$ of CEF cultured in 25 cm² culture bottle. The procedure was repeated 10 times to prepare a recombinant virus stock.

This recombinant virus stock was subjected to plaque assay in a manner similar to Example 1 (5). Red-stained plaques were noted, confirming that lacZ gene was stably retained and expressed therein.

(5) Comparison of HN gene expression between fNZ2637△T and fNZ2637

fNZ2637△T and fNZ2637 were infected to $4 \times 10^4$ of CEF cultured overnight, respectively, at $4 \times 10^4$ PFU. Six hours after the infection, the infected viruses were fixed with cold acetone for 5 minutes and treated using anti-NDV chick antiserum as a first antibody and isothiocyanate fluorescein-bound anti-chick IgG (manufactured by Capel Co.) as a second antibody. Fluorescence microscopic observation indicates that fluorescence in fNZ2637-infected cells is obviously more intense. As the result, it is revealed that transcription termination factor present at the downstream of the gene participates in potentiating expression of the gene.

INDUSTRIAL APPLICABILITY

According to the present invention, virus promoter capable of expressing polypeptide in recombinant virus can be provided and the promoter can be selected, which leads to great progress in the field of manufacturing vaccine. Using these promoters, poxvirus which can stably express an exogenous gene can be constructed so that vaccine can be stably produced.

## Claims

1. A nucleotide sequence containing a part of or all of nucleotide sequence having a promoter activity which is shown in Fig. 1, 2, 3 or 4.

2. A nucleotide sequence according to claim 1, wherein the nucleotide sequence contains a part of or all of a nucleotide sequence from 404 to 515 nucleotides in the nucleotide sequence shown in Fig. 4.

3. A nucleotide sequence according to claim 2, wherein the nucleotide sequence contains a part of or all of a nucleotide sequence from 489 to 505 nucleotides in the nucleotide sequence shown in Fig. 4.

4. A chimera gene comprising a nucleotide sequence of claim 1, 2 or 3 and an exogenous gene encoding polypeptide, said exogenous gene being under control of said nucleotide sequence.

5. A chimera gene according to claim 4, wherein said exogenous gene is a gene encoding Newcastle disease virus antigen.

6. A chimera gene according to claim 5, wherein said Newcastle disease virus antigen is hamagglutinin neuraminidase.

7. A plasmid containing a chimera gene according to any one of claims 4, 5 and 6 and capable of proliferating in Escherichia coli .

8. A plasmid according to claim 7, wherein said chimera gene is inserted in a nucleotide sequence derived from genome region non-essential to proliferation of Avipoxvirus.

9. A recombinant Avipoxvirus comprising a chimera gene according to any one of claims 4, 5 or 6 which is incorporated in a genome region non-essential to proliferation of Avipoxvirus.

10. A recombinant Avipoxvirus according to claim 9, wherein said Avipoxvirus is fowlpoxvirus.

11. A method for selection of a nucleotide sequence having a promoter activity which comprises inserting a virus-derived DNA fragment at the upstream of marker gene of a plasmid containing marker gene under control of no promoter, constructing a recombinant virus using said plasmid, infecting marker gene-deleted cells with the recombinant virus, selecting the cell in which the marker gene has been expressed and, analyzing a nucleotide sequence of the inserted DNA fragment.

12. A method for selection of a nucleotide sequence having promoter activity according to claim 10, wherein said marker gene is lacZ gene.

Amended claims in accordance with Rule 86(2)EPC

1. A nucleotide sequence containing at least a part of the nucleotide sequence having a promoter activity which is shown in Fig. 1, 2, 3 or 4.

2. The nucleotide sequence according to claim 1, which contains at least a part of the nucleotide sequence from nucleotide 404 to 515 in the nucleotide sequence shown in Fig. 4.

3. The nucleotide sequence according to claim 2, which contains at least a part of the nucleotide sequence from nucleotide 489 to 505 in the nucleotide sequence shown in Fig. 4.

4. A chimeric gene comprising a nucleotide sequence of any one of claims 1 to 3 and an exogenous gene encoding a polypeptide, said exogenous gene being under the control of said nucleotide sequence.

5. The chimeric gene according to claim 4, wherein said exogenous gene is a gene encoding a Newcastle disease virus antigen.

6. The chimeric gene according to claim 5, wherein said Newcastle disease virus antigen is haemagglutinin-neuraminidase.

7. A plasmid containing a chimeric gene according to any one of claims 4 to 6 and being capable of proliferating in Escherichia coli .

8. The plasmid according to claim 7, wherein said chimeric gene is inserted into a nucleotide sequence derived from a genomic region being non-essential for the proliferation of Avipoxvirus.

9. A recombinant Avipoxvirus comprising a chimeric gene according to any one of claims 4 to 6 which is incorporated into a genomic region being non-essential for the proliferation of Avipoxvirus.

10. The recombinant Avipoxvirus according to claim 9, wherein said Avipoxvirus is a fowlpoxvirus.

# FIG. I

EP 0 419 666 A1

```
        10          20          30          40          50          60          70
GATCCTCATT  ATATGTTATG  CTATAAATAG  AGATATAAAA  ACAGGGTACG  AGTAAATCGC  TGAGTGTATC

        80          90         100         110         120         130         140
GGCCAGAATA  GTAGATTTAT  TATTGAAATA  AACCATTAAG  TAATATGTAT  TACTCGGCTT  CGGGTACATC

       150         160         170         180         190         200         210
TTTCAATCGA  GTTCTATACG  CAGTAATATC  ACTTTTTTTT  CTTTTAATAG  TAGTAACGTC  AACAGCCAAA

       220         230         240         250         260         270         280
TGCATCATTA  TATGTTCCAC  GGTAATCAAA  TCTATTGGAA  ATTCTATAAA  TAAATCATGT  TTTTCTAACA

       290         300         310         320         330         340         350
TCCATTCTTG  TATGTAGGCT  AGTTCCGAAG  ACGTCATAGG  AACAGTCATT  TTTAATCGTT  TATTAGAACA

       360         370         380         390         400         410         420
TCTACCAAAG  AAGCACTTTC  CGTTTATGAC  AGTGTTGATA  ATATTTTGAC  AGGATGATGG  TAAAGATATC

       430         440         450         460         470         480         490
ATTTCAAAAA  TCTACTTAAA  CATCAATTTT  ATAGAATTAC  TTTTACTTTA  GATTTATAAC  TAGATACAAA

       500
TAAGTATATT  ATAGGATC
```

# FIG. 2

GATCTGATAA TTCTAGATAT TATTAAACTA GTTTTTTTAC TTTTTTTATA TCTATACATC TTTCTGTATT

AAATACTAGG TTGTTATGAA GGTTTATGAA AAAGAAATAT ATATAATTAA TATCGTTACT TGACATAATA

TTGTTCTTCT GTATTGCCTG TAGAGCGTGT TCCTTACATT CAGAGCACGG TAATGCTTTA CATATGCTAT

ACAAGTGACG TTTGCAAGTT TCTATATCGT GTTTGAACTT GGTAATTATA ATAAATATAA CCTAGCCAAA

AGCTGCTTCC CCAGTATCTA GGATC

EP 0 419 666 A1

# FIG. 3

```
          10         20         30         40         50         60         70
GATCCAGTAT TGGTAGAATA TTCGAAAGAT AATGAATGTA AAAAAAATAG TAGCAAATAC AAATACAAAT

          80         90        100        110        120        130        140
TACACTGATG TACTACTACT ACAGTAATAT TTACGGTAAG TTACTTGGGT CTAAGTAGAG ATTATGTAGC

         150        160        170        180        190        200        210
ATCTATGACT TCTTACGATG AAAAATTACA GGACCTTTCT GTGGTTCTGA TCTCTGGATC ATCTTTCATG

         220        230        240        250        260        270        280
GAACCTGGAC AGAAATCTCG TTATAAGAGG TGGTTGGTTT AATTTTTGAT ATTTTTTAA GATTAATACA

         290        300        310        320
TTTTTAATTA AAAAGTGAAT AAGTATATTA ACTACTATAT GTGATC
```

# FIG. 5

```
          10         20         30         40         50         60         70
ATCCTTCCGG AAAACGCAAG AAAAAATTCC GACGGGTGTGC GAGAATGCTG TCCTGTAAGA GGAAGATTGT

          80         90        100        110        120
ACTACGTTAT ATTACTAGAC TGCTGAAGAT ATAATACTAC TAGCTTAATA T
```

22

# FIG. 4

EP 0 419 666 A1

```
     10         20         30         40         50         60         70
GATATCAGAT AAAACCGTAG TAATAAAAGA TTTTTCATAT TGTGACGAAC TATGTCCCGA CGCTATAATA

     80         90        100        110        120        130        140
GGAAGTGTTG TAAAACCGAT CATCATGCGA GTACGTCCAT GGAGATATAT GCGATATATG TGGGTTTGAG

    150        160        170        180        190        200        210
GCTTTACATC CAACCGATAT TGATAAAAGA TTGACTCACG AAAAGGTATG TATGCAACTA CTATGCAAAG

    220        230        240        250        260        270        280
AAGATATAAA ATAGGATAAA TGGGGTATTT GTTTGGATGG TATAAAAGGA AATAAGAAAG CTTATGGTAT

    290        300        310        320        330        340        350
TTTATCAGAC TGTAATCATA TGTTTTGTAT TAACTGTATT AAAACATGGA TGACTACTAT TAATTCTAAG

    360        370        380        390        400        410        420
AAGCAATGCC CTGAATGCAG AGTACCTTCT AAGTATATCA TACAAAGTCC TATCTGGACA GTGGATAAGG

    430        440        450        460        470        480        490
TTAGTAAGAA TCAGTTAAGT GTTTCGTACA AGACTGTATA TATAAAATCA TGTTAACGTT TTGTAGAAGT

    500        510        520        530        540        550        560
TGAAAAAATA ATATACTGAT ACAAAATGGC TTTTCAAGAA CTTTGTTGTA GTAATTTGTT AAAATTCGAG

    570        580        590
AACTGTTCAC TACTCGAAAC GCATAAAAAG ATATC
```

# FIG. 6-1

```
        10         20          30         40          50         60         70
AGATGACCAA AGGGCGATAT ACGGGTAGAA CGGTCGGGGA GGCCGTCCCT CAATCGGGAG TCGGGCCTCA


        80         90         100        110              120        129
CAATATCCGT TCTACCGCAT CACCAATAGC AGTCTTCAGT C ATG GAC CGC GCA GTT AGC
                                             MET Asp Arg Ala Val Ser


        138        147        156        165        174        183
CAA GTT GCG CTA GAG AAT GAT GAA AGA GAG GCA AAG AAT ACA TGG CGC TTG GTA
Gln Val Ala Leu Glu Asn Asp Glu Arg Glu Ala Lys Asn Thr Trp Arg Leu Val


        192        201        210        219        228        237
TTC CGG ATT GCA ATC CTA CTT TTA ACG GTA GTG ACC TTA GCC ATC TCT GCA GCC
Phe Arg Ile Ala Ile Leu Leu Leu Thr Val Val Thr Leu Ala Ile Ser Ala Ala


        246        255        264        273        282        291
GCC CTT GCA TAT AGT ATG GAG GCC AGC ACA CCT AGC GAT CTT GTG GGC ATA CCG
Ala Leu Ala Tyr Ser MET Glu Ala Ser Thr Pro Ser Asp Leu Val Gly Ile Pro


        300        309        318        327        336        345
ACT GCG ATC TCT AGA ACA GAG GAA AAG ATT ACA TCT GCA CTC GGT TCC AAT CAA
Thr Ala Ile Ser Arg Thr Glu Glu Lys Ile Thr Ser Ala Leu Gly Ser Asn Gln


        354        363        372        381        390        399
GAT GTA GTA GAT AGG ATA TAT AAG CAG GTG GCC CTC GAA TCT CCA CTG GCA TTG
Asp Val Val Asp Arg Ile Tyr Lys Gln Val Ala Leu Glu Ser Pro Leu Ala Leu
```

EP 0 419 666 A1

# FIG. 6-2

```
        408            417            426            435            444            453
CTA AAC ACC GAA TCT ACA ATT ATG AAC GCA ATA AAG TCT CTC TCT TAT CAG ATC
Leu Asn Thr Glu Ser Thr Ile MET Asn Ala Ile Lys Ser Leu Ser Tyr Gln Ile

        462            471            480            489            498            507
AAT GGG GCC GCA AAT AGC AGC GGG TGT GGG GCA CCT ATT CAT GAT CCA GAT TAT
Asn Gly Ala Ala Asn Ser Ser Gly Cys Gly Ala Pro Ile His Asp Pro Asp Tyr

        516            525            534            543            552            561
ATT GGA GGA ATA GGT AAA GAA CTT ATT GTA GAT GAT GCT AGC GAC GTC ACA TCA
Ile Gly Gly Ile Gly Lys Glu Leu Ile Val Asp Asp Ala Ser Asp Val Thr Ser

        570            579            588            597            606            615
TTC TAT CCC TCT GCG TTC CAA GAA CAC CTG AAC TTT ATC CCG GCG CCT ACT ACA
Phe Tyr Pro Ser Ala Phe Gln Glu His Leu Asn Phe Ile Pro Ala Pro Thr Thr

        624            633            642            651            660            669
GGA TCA GGT TGC ACT CGG ATA CCC TCA TTT GAC ATG AGC GCT ACC CAC TAC TGT
Gly Ser Gly Cys Thr Arg Ile Pro Ser Phe Asp MET Ser Ala Thr His Tyr Cys

        678            687            696            705            714            723
TAT ACT CAC AAT GTG ATA TTA TCT GGC TGC AGA GAT CAC TCG CAC TCA CAT CAG
Tyr Thr His Asn Val Ile Leu Ser Gly Cys Arg Asp His Ser His Ser His Gln

        732            741            750            759            768            777
TAT TTA GCA CTT GGT GTG CTT CGG ACA TCT GCA ACA GGG AGG GTA TTC TTT TCC
Tyr Leu Ala Leu Gly Val Leu Arg Thr Ser Ala Thr Gly Arg Val Phe Phe Ser
```

EP 0 419 666 A1

# FIG. 6-3

```
        786              795              804              813              822              831
ACT CTG CGT TCC ATC AAT CTG GAT GAC ACC CAA AAT CGG AAG TCT TGC AGT GTG
Thr Leu Arg Ser Ile Asn Leu Asp Asp Thr Gln Asn Arg Lys Ser Cys Ser Val

        840              849              858              867              876              885
AGT GCA ACC CCC TTG GGT TGT GAT ATG CTG TGC TCT AAA GTC ATA GAG ACT GAA
Ser Ala Thr Pro Leu Gly Cys Asp MET Leu Cys Ser Lys Val Ile Glu Thr Glu

        894              903              912              921              930              939
GAA GAG GAT TAT AAC TCA GCT ATC CCC ACG TCG ATG GTA CAT GGA AGG TTA GGG
Glu Glu Asp Tyr Asn Ser Ala Ile Pro Thr Ser MET Val His Gly Arg Leu Gly

        948              957              966              975              984              993
TTC GAC GGC CAA TAC CAC GAG AAA GAC CTA GAT GTC ACA ACA CTA TTC GAG GAC
Phe Asp Gly Gln Tyr His Glu Lys Asp Leu Asp Val Thr Thr Leu Phe Glu Asp

        1002             1011             1020             1029             1038             1047
TGG GTG GCA AAC TAC CCA GGA GTA GGG GGC GGA TCT TTT ATT GAC AAC CGC GTA
Trp Val Ala Asn Tyr Pro Gly Val Gly Gly Gly Ser Phe Ile Asp Asn Arg Val

        1056             1065             1074             1083             1092             1101
TGG TTC CCA GTT TAC GGA GGG CTA AAA CCC AAT TCG CCC AGT GAC ACC GCA CAA
Trp Phe Pro Val Tyr Gly Gly Leu Lys Pro Asn Ser Pro Ser Asp Thr Ala Gln
```

EP 0 419 666 A1

# FIG. 6-4

```
        1110            1119            1128            1137            1146            1155
GAA GGG AAA TAT GTA ATA TAC AAG CGA TAC AAT GAC ACA TGT CCA GAT GAG CAG
Glu Gly Lys Tyr Val Ile Tyr Lys Arg Tyr Asn Asp Thr Cys Pro Asp Glu Gln

        1164            1173            1182            1191            1200            1209
GAT TAT CAG ATT CGA ATG GCT AAG TCT TCA TAT AAG CCT GGG CGA TTT GGT GGG
Asp Tyr Gln Ile Arg MET Ala Lys Ser Ser Tyr Lys Pro Gly Arg Phe Gly Gly

        1218            1227            1236            1245            1254            1263
AAA CGA GTA CAG CAG GCC ATC TTA TCT ATC AAA GTG TCA ACA TCC TTG GGC GAG
Lys Arg Val Gln Gln Ala Ile Leu Ser Ile Lys Val Ser Thr Ser Leu Gly Glu

        1272            1281            1290            1299            1308            1317
GAC CCG GTG CTG ACT GTG CCG CCC AAC ACA GTC ACA CTC ATG GGG GCC GAA GGC
Asp Pro Val Leu Thr Val Pro Pro Asn Thr Val Thr Leu MET Gly Ala Glu Gly

        1326            1335            1344            1353            1362            1371
AGA GTT CTC ACA GTA GGG ACA TCT CAT TTC TTT TAT CAG CGA GGG TCG TCA TAC
Arg Val Leu Thr Val Gly Thr Ser His Phe Phe Tyr Gln Arg Gly Ser Ser Tyr

        1380            1389            1398            1407            1416            1425
TTC TCC CCT GCC CTA CTA TAT CCT ATG ACA GTC AGC AAC AAA ACA GCT ACT CTT
Phe Ser Pro Ala Leu Leu Tyr Pro MET Thr Val Ser Asn Lys Thr Ala Thr Leu

        1434            1443            1452            1461            1470            1479
CAT AGT CCT TAT ACA TTC AAT GCC TTC ACT CGA CCA GGT AGT GTC CCC TGC CAG
His Ser Pro Tyr Thr Phe Asn Ala Phe Thr Arg Pro Gly Ser Val Pro Cys Gln

        1488            1497            1506            1515            1524            1533
GCT TCA GCA AGA TGC CCT AAC TCA TGT GTT ACC GGA GTC TAT ACT GAT CCA TAT
Ala Ser Ala Arg Cys Pro Asn Ser Cys Val Thr Gly Val Tyr Thr Asp Pro Tyr
```

EP 0 419 666 A1

# FIG. 6-5

```
        1542          1551          1560          1569          1578          1587
CCC TTG GTC TTT TAT AGG AAC CAC ACC TTG CGA GGG GTA TTC GGG ACG ATG CTT
Pro Leu Val Phe Tyr Arg Asn His Thr Leu Arg Gly Val Phe Gly Thr MET Leu


        1596          1605          1614          1623          1632          1641
GAT GAT GAA CAA GCA AGA CTT AAC CCT GTA TCT GCA GTA TTT GAC AGC ATA TCT
Asp Asp Glu Gln Ala Arg Leu Asn Pro Val Ser Ala Val Phe Asp Ser Ile Ser


        1650          1659          1668          1677          1686          1695
CGC AGT CGC ATA ACC CGG GTG AGT TCA AGC AGC ACC AAG GCA GCA TAC ACA ACA
Arg Ser Arg Ile Thr Arg Val Ser Ser Ser Ser Thr Lys Ala Ala Tyr Thr Thr

                                                                            *
        1704          1713          1722          1731          1740          1749
TCA ACA TGT TTT AAA GTT GTA AAG ACC AAT AAG ACA TAT TGT CTC AGC ATT GCC
Ser Thr Cys Phe Lys Val Val Lys Thr Asn Lys Thr Tyr Cys Leu Ser Ile Ala


        1758          1767          1776          1785          1794          1803
GAA ATA TCC AAT ACC CTC TTC GGG GAA TTC AGA ATC GTC CCT TTA CTA GTT GAG
Glu Ile Ser Asn Thr Leu Phe Gly Glu Phe Arg Ile Val Pro Leu Leu Val Glu


        1812          1821          1830          1839          1848          1857
ATT CTC AAG GAT GAT GGG GTT AGA GAA GCC AGG TCT GGC CGG TTG AGT CAA TTG
Ile Leu Lys Asp Asp Gly Val Arg Glu Ala Arg Ser Gly Arg Leu Ser Gln Leu


        1866          1875          1884          1893          1902          1911
CAA GAG GGT TGG AAA GAT GAC ATT GTA TCG CCT ATC TTT TGC GAC GCC AAG AAT
Gln Glu Gly Trp Lys Asp Asp Ile Val Ser Pro Ile Phe Cys Asp Ala Lys Asn


        1920          1929          1938          1947          1956          1969
CAA ACT GAG TAC CGG CGC GAG CTC GAG TCC TAC GCT GCC AGC TGG CCA TAATCAGCTA
Gln Thr Glu Tyr Arg Arg Glu Leu Glu Ser Tyr Ala Ala Ser Trp Pro


        1979          1989          1999
GTGCTAATGT GATTAGATTA AGTCTCGTCG A
```

EP 0 419 666 A1

## FIG. 7

pNZ131

7.3 kbp

V V
Hp
H
Hp
K
H
Hp
E
K
B E
pUC18
2.7 kbp

ℓacZ — ℓac Z GENE

VECTOR-DERIVED DNA

NP STRAIN DNA

B: BamHI

H: HindⅢ

E: EcoRI

Hp: HpaI

P: PvuⅡ

V: EcoRⅤ

K: KpnI

## FIG. 8

pNZ1127R

3.2kbp
P E P ℓacZ
(B)
V
P B
V
P
4.4kbp
2.9kbp
E P
E
P
pUC18
2.7kbp

# FIG. 9

# FIG. 10

# FIG. II

# FIG. 12

EP 0 419 666 A1

# FIG. 13

# FIG. 14

B: BamHI
E: EcoRI
H: HindIII
Bg: BglII
S: SspI

*lacZ* : *lac* Z GENE
HN : HN GENE
: NP STRAIN DNA

# FIG. 15

```
Sau : Sau3AI
B : BamHI
S : SspI
H : HindⅢ
E : EcoRI
lacZ : lac Z GENE
HN : HN GENE
: NP STRAIN DNA
```

# FIG. 16

```
        10          20          30          40
AACGTTTTGT AGAAGTTGAA AAAATAATAT ACTGATACAA ACTGCA
TTGCAAAACA TCTTCAACTT TTTTATTATA TGACTATGTT TG
```

36

EP 0 419 666 A1

# FIG. 17

# F I G. 18

B : BamHI

Sa : Sac I

C : Cℓa I

K : Kpn I

ℓacZ
[ ] : ℓac Z GENE

HN
[▒▒▒] : HN GENE

[////] : NP STRAIN DNA

[ P ] : 7.5K PROMOTER

# FIG. 19

Sa : Sac I
C : Cℓa I
K : Kpn I

lacZ
▭ : ℓac Z GENE

HN
◼ : HN GENE

▨ : NP STRAIN DNA

P : 7.5 K PROMOTER

# INTERNATIONAL SEARCH REPORT

International Application No PCT/JP89/01330

## I. CLASSIFICATION OF SUBJECT MATTER (if several classification symbols apply, indicate all) 6

According to International Patent Classification (IPC) or to both National Classification and IPC

Int. Cl$^5$    C12N15/63, 15/83, C12N7/01

## II. FIELDS SEARCHED

| Minimum Documentation Searched 7 | |
|---|---|
| Classification System | Classification Symbols |
| IPC | C12N7/00 - 7/08, 15/00 - 15/90 |

| Documentation Searched other than Minimum Documentation to the Extent that such Documents are Included in the Fields Searched 8 |
|---|
| BIOSIS Data Base |

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| A | WO, A1, 88/02022 (Commonwealth Scientific And Industrial Research Organization), 24 March 1988 (24. 03. 88) | 1 - 10 |
| A | EP, A1, 284416 (Nippon Zeon Co., Ltd.), 28 September 1988 (28. 09. 88) | 1 - 10 |
| X | JP, A, 61-170393 (Nippon Zeon Co., Ltd.), 1 August 1986 (01. 08. 86), (Family: none) | 11 - 12 |
| X | JP, A, 61-271990 (Kogyo Gijutsuin-cho), 2 December 1986 (02. 12. 86), (Family: none) | 11 - 12 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| March 26, 1990 (26. 03. 90) | April 16, 1990 (16. 04. 90) |
| International Searching Authority | Signature of Authorized Officer |
| Japanese Patent Office | |